# EUROPÄISCHE PATENTSCHRIFT

(11) **EP 2 177 154 B1**
(45) Veröffentlichungstag und Bekanntmachung des Hinweises auf die Patenterteilung: **04.05.2011**
(21) Anmeldenummer: 08166805.5
(22) Anmeldetag: 16.10.2008
(51) Int. Cl.: A61B 5/00, A63H 3/00

(54) **Kinderfreundliches Blutglucosemessgerät**
Child-friendly blood glucose measuring device
Appareil de mesure de glycémie adapté aux enfants

(43) Veröffentlichungstag der Anmeldung: 21.04.2010
(73) Patentinhaber: Roche Diagnostics GmbH, 68305 Mannheim (DE); F. Hoffmann-La Roche AG, 4070 Basel (CH)
(72) Erfinder: Klinke, Martin, 06110 Halle (DE); Hofmann, Dieter, 06108 Halle (DE)
(74) Vertreter: Stößel, Matthias

(56) Entgegenhaltungen:
- WO-A-03/034912
- WO-A-2008/037061
- US-A- 6 165 035
- US-A1- 2005 176 461

## Beschreibung

Die Erfindung betrifft ein Messsystem zum Nachweis mindestens eines Analyten in einer Körperflüssigkeit. Weiterhin betrifft die Erfindung ein Spielzeug, insbesondere ein Spieltier, welches in dem Messsystem zum Einsatz gelangen kann, sowie ein Kit zum Nachweis mindestens eines Analyten und ein Verfahren zur Herstellung eines Messsystems zum Nachweis mindestens eines Analyten in einer Körperflüssigkeit. Derartige Messsysteme, Spielzeuge, Kits und Verfahren können insbesondere im Bereich der Messung von Blutglucose zum Einsatz kommen. Auch andere Einsatzgebiete sind jedoch möglich.

Zur Gesundheitsvorsorge sowie zur Kontrolle und Behandlung unterschiedlicher Arten von Erkrankungen ist in vielen Fällen ein qualitativer und/oder quantitativer Nachweis verschiedener Analyte in Körperflüssigkeiten eines menschlichen oder tierischen Patienten erforderlich. Die vorliegende Erfindung betrifft insbesondere die Überwachung von Blutglucose. Alternativ oder zusätzlich ist die Erfindung jedoch auch in anderen Bereichen einsetzbar, beispielsweise für den qualitativen und/oder quantitativen Nachweis anderer Arten von Analyten. So lassen sich beispielsweise Cholesterin und/oder Lactat nachweisen, oder es lassen sich Koagulationsmessungen durchführen. Auch andere Arten von Körperflüssigkeiten als Blut kommen in Betracht, beispielsweise Urin oder interstitielle Flüssigkeit. Ohne Beschränkung weiterer möglicher Ausführungsformen und Einsatzgebiete wird die Erfindung im Folgenden am Beispiel der Blutglucosemessung beschrieben.

Für den Nachweis des Analyten werden dabei in vielen Fällen Messgeräte verwendet, welche den Analyten mittels Testelementen nachweisen. Diese Testelemente können beispielsweise als Teststreifen, Testbänder, Testscheiben, faltbare Testelemente oder in anderer Form vorliegen und können beispielsweise als Mehrweg- oder als Einmal-Testelemente (Disposables) ausgestaltet sein. Neben Messgeräten, welche mit Einzelteststreifen arbeiten, sind Messgeräte bekannt, welche mit einer Mehrzahl von magazinierten Testelementen arbeiten. Der Nachweis des mindestens einen Analyten kann beispielsweise auf elektrochemischem Wege und/oder unter Verwendung optischer Messmethoden erfolgen. In der Regel weisen die Testelemente mindestens ein Testfeld mit einer Testchemie auf, welche mindestens eine nachweisbare physikalische und/oder chemische Eigenschaft bei Anwesenheit des mindestens einen Analyten ändern. Zahlreiche Arten von Messverfahren, Testelementen und Messgeräten sind bekannt, auf welche auch im Rahmen der vorliegenden Erfindung zurückgegriffen werden kann.

Für eine Diabeteskontrolle im Bereich des so genannten "Home Monitoring" sind in der Regel bis zu sieben Messungen täglich erforderlich. Um den Tagesablauf des Patienten durch diese Messungen nicht unnötig einzuschränken, sind Messgeräte in der Regel als tragbare Messgeräte ausgestaltet, so dass diese beispielsweise durch einen Patienten am Arbeitsplatz oder in der Freizeit mitgeführt werden können. Da die erforderlichen Messungen in vielen Fällen mit einer vorgegebenen Regelmäßigkeit durchgeführt werden müssen, ist dennoch ein erhebliches Maß an Disziplin erforderlich, um eine lückenlose Überwachung gewährleisten zu können.

Eine derartige Sorgfalt und Disziplin ist jedoch keine Selbstverständlichkeit, sondern muss erlernt werden und ist in den meisten Fällen das Ergebnis einer sorgsamen Erziehung. Eine kleine, jedoch nicht zu vernachlässigende Patientengruppe stellen dabei im Bereich der Diabeteskontrolle Kinder und Minderjährige dar, insbesondere bei Diabetes Typ I, jedoch in zunehmendem Maße auch bei Typ II. Gerade bei Kindern und Jugendlichen besteht jedoch eine erhebliche Schwierigkeit darin, diese Patienten zu einem regelmäßigen Messverhalten anzuhalten.

Aus dem Stand der Technik sind daher Systeme bekannt, welche speziell für Patienten im Kindesalter konstruiert sind. So beschreibt beispielsweise WO 03/034912 A1 eine Simulationsvorrichtung zur spielerischen Auswertung und Anzeige von Blutglucosewerten. Dabei erfolgt die Anzeige der Auswertung mittels einer virtuellen Kreatur, welche auf einem Display dargestellt wird. Diese virtuelle Kreatur kann mit dem Anwender auf verschiedene Arten kommunizieren. Das Gehäuse der Simulationsvorrichtung kann beispielsweise in Form eines Teddybären ausgestaltet sein.

Derartige, speziell auf Patienten im Kindesalter zugeschnittene Geräte weisen jedoch in der Praxis erhebliche Nachteile auf. Insbesondere ist hierbei der in den letzten Jahren erheblich gestiegene Kostendruck im Gesundheitswesen zu nennen. Messgeräte müssen aufgrund dieses Kostendrucks in der Regel nahezu kostenneutral hergestellt werden. Die genannten, Kinder-spezifischen Geräte müssen jedoch für einen vergleichsweise kleinen Patientenkreis speziell und separat hergestellt werden. Auch innerhalb dieses Patientenkreises können dabei keine einheitlichen Geräte verwendet werden, da, je nach angesprochener Altersstufe der Patienten, die Geräte in ihrer äußeren Erscheinungsform und/oder in ihrer Software angepasst werden müssen.

Es ist ohne weiteres ersichtlich, dass die bekannten Spezialanfertigungen für einen kleinen Kundenkreis mit erheblichem Aufwand hinsichtlich Produktion, Lagerhaltung und Logistik verbunden sind. Zudem ist zu beachten, dass das Design von für Kinder oder Jugendliche bestimmten Produkten in hohem Maße Modeerscheinungen unterworfen ist, was beispielsweise durch die Medien noch gefördert wird. Somit ist nicht nur eine Anpassung des Erscheinungsbildes der Messgeräte an bestimmte Altersstufen erforderlich, sondern auch an derartige Modeerscheinungen, um das Interesse der Kinder und jugendlichen Patienten aufrechtzuerhalten. Diese Schwierigkeiten insgesamt haben dazu geführt, dass sich kindgerechte Messgeräte bislang auf dem Markt nicht in größeren Stückzahlen durchsetzen konnten.

Es ist daher eine Aufgabe der vorliegenden Erfindung, ein Messsystem bereitzustellen, welches die Nachteile bekannter Messsysteme vermeidet. Insbesondere soll das Messsystem kindgerechte Messungen ermöglichen und dennoch kostengünstig auch in hohen Stückzahlen herstellbar sein.

Diese Aufgabe wird durch ein Messsystems, ein Kit sowie ein Verfahren mit den Merkmalen der unabhängigen Ansprüche gelöst. Vorteilhafte Weiterbildungen der Erfindung, welche einzeln oder in Kombination realisierbar sind, sind in den abhängigen Ansprüchen dargestellt. In einem ersten Aspekt der vorliegenden Erfindung wird ein Messsystem zum Nachweis mindestens eines Analyten in einer Körperflüssigkeit vorgeschlagen. Wie oben dargestellt, wird das Messsystem im Folgenden im Wesentlichen in Bezug auf den Nachweis von Blutglucose beschrieben. Auch eine Messung anderer Arten von Analyten und/oder ein Einsatz bei anderen Arten von Körperflüssigkeiten sind jedoch grundsätzlich denkbar.

Dementsprechend umfasst das Messsystem mindestens ein Messgerät zum Nachweis des Analyten in der Körperflüssigkeit. Das Messgerät ist als tragbares Handgerät ausgestaltet und weist ein Gehäuse mit mindestens einem Anzeigenelement auf. Unter einem Messgerät wird dabei allgemein ein Messgerät verstanden, welches den qualitativen und/oder quantitativen Nachweis des mindestens einen Analyten in der Körperflüssigkeit durchführen kann. Insbesondere kann es sich dabei um eines der oben beschriebenen Messgeräte handeln, also ein Messgerät mit einer Messfunktion, mittels derer beispielsweise auf elektrochemischem und/oder optischem Wege der Analytnachweis durchgeführt werden kann. Insbesondere kann das Messgerät, wie oben dargestellt, unter Verwendung mindestens eines Testelements arbeiten, wobei grundsätzlich sämtliche Arten bekannter Testelemente einsetzbar sind. Diesbezüglich kann beispielsweise auf den Stand der Technik verwiesen werden.

Unter einem Anzeigenelement wird dabei allgemein ein Element verstanden, welches eingerichtet ist, um mindestens eine Information an einen Benutzer und/oder Patienten zu übermitteln. Dabei kann es sich beispielsweise um eine optische Information und/oder eine akustische Information und/oder eine haptische Information handeln. Auch andere Arten von Informationen und/oder Kombinationen der genannten Informationen und/oder anderer Arten von Informationen sind jedoch grundsätzlich verwendbar. Besonders bevorzugt ist, wenn das Anzeigenelement als optisches Anzeigenelement ausgestaltet ist und beispielsweise ein Display umfasst.

Um das Messsystem kindgerecht auszugestalten, umfasst das Messsystem weiterhin mindestens ein Spielzeug. Dieses Spielzeug kann grundsätzlich beliebig ausgestaltet sein und kann insbesondere eine Spielfigur und/oder ein Spieltier, vorzugsweise ein Stofftier, umfassen bzw. als ein derartiges Spieltier ausgestaltet sein. Allgemein ist es bevorzugt, wenn das Spielzeug als Figur, insbesondere als menschliche Figur, als tierische Figur oder als Fantasiefigur, ausgestaltet ist, also als Wesen, welchem - zumindest in der Fantasie eines Kindes- ein eigener Charakter zugewiesen werden kann. Eine derartige Ausgestaltung als Figur erleichtert die Kommunikation zwischen dem Patienten und dem Messgerät, da der Umgang mit einer Figur im Allgemeinen zwangloser und weniger formell gestaltet werden kann als mit einer Maschine. Zudem wird einem Patienten im Kindesalter durch diese Ausgestaltung die Angst vor einer Messung zumindest teilweise genommen.

Um die oben beschriebenen Nachteile bekannter Geräte zu vermeiden, wird im Rahmen der vorliegenden Erfindung vorgeschlagen, das Spielzeug mit mindestens einer Aufnahme zu versehen. In dieser Aufnahme soll das Messgerät zumindest reversibel zumindest teilweise aufnehmbar bzw. aufgenommen sein. Unter reversibel ist dabei eine Aufnahme zu verstehen, welche mit wenigen Handgriffen, vorzugsweise auch durch einen Patienten selbst, ein Einlegen und/oder Herausnehmen des Messgeräts ermöglicht. Dabei soll die Aufnahme derart eingerichtet sein, dass bei in der Aufnahme aufgenommenem Messgerät zumindest das Anzeigenelement des Messgeräts zumindest teilweise zugänglich ist. Weiterhin können auch weitere Bereiche und/oder Elemente des Messgeräts zumindest teilweise zugänglich sein.

Dabei ist das Messgerät, insbesondere durch entsprechende Ausgestaltung des Gehäuses, derart eingerichtet, dass das Messgerät sowohl in dem Messsystem als auch wahlweise unabhängig von dem Spielzeug bzw. dem Messsystem einsetzbar ist. Insofern wird vorgeschlagen, insbesondere handelsübliche Messgeräte, welche auch unabhängig von dem Messsystem einsetzbar sind, in dem Messsystem zu verwenden. Diese Verwendung eines unabhängigen Messgeräts kann insbesondere dadurch gewährleistet sein, dass das Gehäuse des Messgeräts derart ausgestaltet ist, dass dieses den Anforderungen an ein Gehäuse eines tragbaren Messgeräts genügt. Beispielsweise kann das Gehäuse eine mechanische Stabilität gewährleisten, welche auch eine Mitnahme des Messgeräts, unabhängig vom Spielzeug, in beispielsweise einer Tasche oder auch ohne Hülle ermöglicht, ohne dass eine mechanische Beschädigung des Messgeräts unter üblichen Bedingungen auftritt. Auch ein Schutz gegenüber Feuchtigkeit und/oder Verschmutzungen kann durch das Gehäuse gewährleistet sein. Insofern kann das Messgerät beispielsweise ein handelsübliches, herkömmliches Messgerät sein.

Die erfindungsgemäße Ausgestaltung des Messsystems mit dem Messgerät und dem Spielzeug mit einer entsprechenden Aufnahme bietet gegenüber bekannten Geräten, welche kindgerecht ausgestaltet sind, zahlreiche Vorteile. So ist in dem Messsystem nicht, wie beispielsweise in WO 03/034912 A1 die Außenhülle des Messgeräts selbst als Spielzeug ausgestaltet, sondern das Messgerät ist in einem derartigen Spielzeug mittels einer geeigneten Aufnahme reversibel aufgenommen. Hierdurch kann das Messgerät gleichzeitig ohne Spielzeug für einen erwachsenen Kundenkreis angeboten werden, ohne dass hierfür Änderungen am Messgerät selbst vorgenommen werden müssen. Durch die Erfindung ist es folglich möglich, eine individuelle, kindgerechte Anpassung eines Messgeräts, beispielsweise eines Blutzuckermessgeräts, vorzunehmen, ohne hierfür Änderungen am Analysesystem selbst durchführen zu müssen.

Die Herstellung derartiger Messsysteme vereinfacht sich dementsprechend erheblich. So wird ein Verfahren zur Herstellung eines Messsystems zum Nachweis mindestens eines Analyten in einer Körperflüssigkeit vorgeschlagen, insbesondere eines Messsystems gemäß der obigen Beschreibung und/oder gemäß einer der nachfolgend noch beschriebenen Ausführungsformen. Auf diese optionalen Ausführungsformen kann dementsprechend für weitere Details verwiesen werden. Dabei wird bei dem vorgeschlagenen Verfahren wiederum ein Messgerät, beispielsweise ein Messgerät gemäß der obigen Beschreibung, verwendet, welches zum Nachweise des Analyten in der Körperflüssigkeit einsetzbar ist. Insbesondere wird wiederum vorgeschlagen, ein handelsübliches Messgerät zu verwenden, welches als tragbares Handgerät ausgestaltet ist und sowohl in dem Messsystem als auch wahlweise unabhängig von dem Messsystem bzw. dem Spielzeug einsetzbar ist. Das Messgerät weist wiederum ein Gehäuse mit mindestens einem Anzeigenelement auf. Weiterhin wird bei dem vorgeschlagenen Verfahren ein Spielzeug verwendet, insbesondere ein Spieltier, wobei das Messgerät in mindestens einer Aufnahme des Spieltiers reversibel aufgenommen wird. Die Aufnahme ist eingerichtet, dass bei in der Aufnahme aufgenommenem Messgerät das Anzeigenelement zumindest teilweise zugänglich ist.

Bei dem erfindungsgemäßen Verfahren kann insbesondere in einer Lagerhaltung eine Mehrzahl unterschiedlicher Arten von Spielzeugen vorgehalten werden. Beispielsweise können unterschiedliche Spielzeuge für unterschiedliche Altersgruppen vorgehalten werden, geschlechtsspezifische Spielzeuge (beispielsweise Figuren, Puppen und/oder Stofftiere), unterschiedliche Regionen, unterschiedliche Modeerscheinungen oder Ähnliches. Die Lagerhaltung ist entsprechend auf aktuelle Vorlieben der Zielgruppen anpassbar und aktualisierbar, ohne dass hierfür eine Anpassung der Messgeräte erforderlich wäre. Bei der Herstellung kann dann, beispielsweise je nach Zielgruppe der Benutzer, eine bestimmte Art von Spielzeug für die Herstellung des Messsystems ausgewählt werden.

Das vorgeschlagene Messsystem und das vorgeschlagene Verfahren führen somit dazu, dass eine kostengünstige Herstellung Zielgruppen-spezifischer Messsysteme möglich ist, welche kindgerecht ausgestaltet werden können und welche auch auf aktuelle Entwicklungen ohne größeren Aufwand angepasst werden können. Hierdurch lassen sich die Kosten erheblich senken, und es lässt sich die Herstellung spezieller Geräte für einen kleinen Kundenkreis vermeiden. Falls erforderlich, können lediglich kleinere Anpassungen vorgenommen werden, beispielsweise Softwareanpassungen des Messsystems. Beispiele derartiger Anpassungen, um beispielsweise den Charakter einer durch das Messsystem dargestellten Figur zu ändern, werden unten beschrieben.

Neben dem Messsystem und dem Herstellungsverfahren wird weiterhin ein Kit zum Nachweis mindestens eines Analyten in der Körperflüssigkeit vorgeschlagen, insbesondere zum Nachweis von Blutglucose. Das Kit umfasst ein Messgerät mit den sich auf ein Messgerät beziehenden, oben beschriebenen Merkmalen. Insbesondere kann es sich dabei wiederum um ein handelsübliches Messgerät handeln. Weiterhin umfasst das Kit eine Mehrzahl von Spielzeugen, insbesondere Spielfiguren und/oder Spieltieren, welche jeweils mindestens eine Aufnahme zum Aufnehmen des Messgeräts aufweisen. Bezüglich weiterer Merkmale dieser Spielzeuge kann wiederum auf die obige Beschreibung sowie auf die noch folgenden möglichen Ausgestaltungen derartiger Spielzeuge verwiesen werden. Dieses Kit kann beispielsweise in der Lagerhaltung eingesetzt werden, um schnell ein kundenspezifisches Messsystem, beispielsweise gemäß der obigen oder der noch folgenden Beschreibung zusammenzustellen. Alternativ kann ein derartiges Kit jedoch auch von einem Kunden direkt eingesetzt werden, beispielsweise um das Erscheinungsbild des Messsystems nach Belieben im Rahmen der im Kit vorgehaltenen Spielzeuge zu verändern. So kann beispielsweise ein Elternteil im Rahmen eines Kits mehreren Kindern individuell Messsysteme bereitstellen, welche an die Vorlieben des jeweiligen Kindes angepasst sind und welche dennoch auf der Verwendung desselben Messgerätes beruhen. Das Kit kann also zur Herstellung eines Messsystems eingesetzt werden. Auf diese Weise lassen sich ebenfalls erhebliche Kosten einsparen, da mehrere Benutzer mit unterschiedlichen Vorlieben auf dasselbe Messgerät zurückgreifen können.

Ein Spielzeug, insbesondere eine Spielfigur oder ein Spieltier, welches in einem erfindungsgemäßen Messsystem zum Einsatz kommen kann, weist mindestens eine, die äußere Gestalt des Spielzeuges im Wesentlichen bestimmende Spielzeughülle auf. Weiterhin umfasst das Spielzeug mindestens eine Aufnahme zum reversiblen Aufnehmen eines Messgeräts zum Nachweis eines Analyten in einer Körperflüssigkeit, insbesondere eines handelsüblichen Messgeräts. Das Messgerät, für dessen mögliche Ausgestaltungen beispielsweise auf die obige oder noch folgende Beschreibung verwiesen werden kann, ist als tragbares Handgerät ausgestaltet und weist ein Gehäuse mit mindestens einem Anzeigenelement auf. Die Aufnahme des Spielzeugs ist dabei derart eingerichtet, dass bei in der Aufnahme auf genommenem Messgerät das Anzeigenelement zumindest teilweise zugänglich ist.

Ein derartig ausgestaltetes Spielzeug, welches speziell durch die Aufnahme für die Verwendung in einem erfindungsgemäßen Messsystem ausgestaltet ist, kann beispielsweise als eigenständiges Produkt vertrieben werden, unabhängig vom Messgerät. So kann beispielsweise auf Änderungen in den Vorlieben der Zielgruppe reagiert werden. Elternteile können beispielsweise, wenn sich die Vorlieben der Kinder verändern, auf diese geänderten Vorlieben durch den Kauf neuer, aktualisierter oder geänderter Spielzeuge reagieren. Gleichzeitig können Hersteller durch den unabhängigen Vertrieb derartiger Spielzeuge auf Modeerscheinungen eingehen, so dass, auch bei gleichbleibendem Design des Messgeräts, eine Aktualisierung des äußeren Erscheinungsbilds der Messsysteme möglich ist. Auch hierdurch lassen sich erhebliche logistische Einsparungen erzielen.

Das Anzeigenelement soll, wie oben dargestellt, vorzugsweise als optisches Anzeigenelement ausgestaltet sein und kann beispielsweise mindestens ein Display umfassen. Alternativ oder zusätzlich sind jedoch auch andere Arten von Anzeigenelementen möglich. Beispielsweise können Leuchtdioden, Leuchtflächen oder ähnliche Arten optischer Anzeigenelemente eingesetzt werden. Weiterhin weist das Messgerät zusätzliche Elemente auf, die eine Interaktion zwischen Benutzer und Messgerät gewährleisten. So weist das Messgerät weiterhin mindestens ein Bedienelement auf, wobei die Aufnahme vorzugsweise derart eingerichtet ist, dass bei in der Aufnahme aufgenommenem Messgerät auch das Bedienelement zumindest teilweise zugänglich ist. Unter "zugänglich" ist dabei bezüglich des Anzeigenelements zu verstehen, dass die bereitgestellten, beispielsweise angezeigten, Informationen auch bei in der Aufnahme aufgenommenem Messgerät wahrgenommen werden können. Beispielsweise kann ein Display nicht oder lediglich teilweise durch das Spielzeug, beispielsweise eine Spielzeughülle, bedeckt sein. Hinsichtlich der Bedienelemente bedeutet der Begriff "zugänglich", dass die Bedienung dieser Bedienelemente durch den Benutzer bzw. Patienten zumindest eingeschränkt, vorzugsweise uneingeschränkt, auch bei in der Aufnahme aufgenommenem Messgerät möglich ist. Das mindestens eine Bedienelement kann beispielsweise Druckknöpfe, Drehknöpfe, Tasten oder Ähnliches umfassen. Alternativ oder zusätzlich kann das mindestens eine Bedienelement auch ganz oder teilweise mit dem Anzeigenelement kombiniert sein, beispielsweise indem ein Touch-Screen verwendet wird, auf welchem beispielsweise virtuelle Bedienelemente angezeigt werden.

Die Aufnahme kann speziell auf das Messgerät angepasst sein. Diese Anpassung kann beispielsweise dadurch erfolgen, dass die Aufnahme speziell auf die Dimensionen eines bestimmten Typs oder einer Gruppe von Typen von Messgeräten dimensioniert ist. Auch eine Ausgestaltung der Aufnahme für mehrere Arten von Messgeräten ist denkbar.

Um das Messgerät in der Aufnahme zu fixieren, weist Aufnahme weiterhin mindestens eine Fixiervorrichtung auf, welche eingerichtet ist, um das Messgerät relativ zu dem Spielzeug zu fixieren. Insbesondere kann das Spielzeug beispielsweise eine Öffnung aufweisen. Diese Öffnung, wobei sinngemäß auch mehrere Öffnungen vorgesehen sein können, kann beispielsweise die Zugänglichkeit zu dem mindestens einen Anzeigenelement und/oder dem mindestens einen Bedienelement gewährleisten. Dementsprechend kann unter einer Öffnung nicht nur eine vollständig unbedeckte Öffnung verstanden werden, sondern es sind beispielsweise auch Öffnungen denkbar, welche eine optische Zugänglichkeit ermöglichen und dementsprechend ganz oder teilweise mit einem transparenten Material, beispielsweise einem transparenten Kunststoff, bedeckt sind. Die Fixiervorrichtung kann beispielsweise eingerichtet sein, um das Messgerät derart zu fixieren, dass durch die mindestens eine Öffnung der Zugang auch unter üblichen Belastungen eines Spielzeugs erhalten bleibt. Insbesondere kann auf diese Weise ein Verrutschen oder Verkippen des Messgeräts relativ zu der Öffnung verhindert werden.

Die Fixiervorrichtung kann beispielsweise eine Klemmvorrichtung, eine Schraubvorrichtung, eine Schiene oder eine beliebige andere Art von Fixiervorrichtung umfassen, welche üblicherweise für die Fixierung von Geräten eingesetzt werden und welche dem Fachmann bekannt sind. Die Fixiervorrichtung kann mit einer entsprechenden Fixiervorrichtung am Messgerät zusammenwirken und eine gemeinsame Fixiervorrichtung bilden. Alternativ können jedoch auch Fixiervorrichtungen vorgesehen sein, welche unabhängig von entsprechenden Gegenstücken am Messgerät auskommen, so dass vorzugsweise handelsübliche Messgeräte eingesetzt werden können, welche nicht speziell auf das Messsystem angepasst sind.

Die Aufnahme kann insbesondere einen Aufnahmeraum umfassen. In diesem Aufnahmeraum kann das Messgerät zumindest teilweise aufnehmbar sein. Weiterhin kann die Aufnahme mindestens ein Verschlusselement aufweisen, welches auch ganz oder teilweise identisch mit der Fixiervorrichtung sein kann. Dieses Verschlusselement kann dann eingerichtet sein, um das Messgerät in einem geschlossenen Zustand in dem Aufnahmeraum zu haltern und in einem geöffneten Zustand ein Herausnehmen des Messgeräts aus der Auf nahme zu ermöglichen. Das Verschlusselement kann insbesondere derart eingerichtet sein, dass ein Öffnen und Schließen des Verschlusselements auch durch einen Benutzer, insbesondere durch ein Elternteil und/oder einen Benutzer im Kindesalter, möglich ist. Auf diese Weise lassen sich die oben dargestellten Vorteile der Austauschbarkeit des Spielzeugs in dem Messsystem besonders einfach gewährleisten. Diese Austauschbarkeit kann dann nicht nur während einer Produktion, sondern beispielsweise auch im täglichen Gebrauch gewährleistet sein, im Gegensatz beispielsweise zu Systemen wie dem in WO 03/034912 A1 beschriebenen System, bei welchem ein Austausch des äußeren Erscheinungsbildes in der Regel nur durch einen Fachmann mit entsprechenden Werkzeugen möglich ist. Das erfindungsgemäße Verschlusselement kann insbesondere derart eingerichtet sein, dass ein Öffnen und Schließen dieses Verschlusselements ohne zusätzliche Werkzeuge möglich ist. Beispielsweise kann das Verschlusselement mindestens eines der folgenden Elemente umfassen: einen Reißverschluss; einen Druckknopf; einen Textilknopf; eine durch einen Benutzer lösbare Sperre.

Wie oben dargestellt ist das Messgerät insbesondere eingerichtet, um den Analyten mittels mindestens eines Testelements nachzuweisen. Sämtliche aus dem Stand der Technik bekannte Arten von Testelementen sind dabei grundsätzlich auch im Rahmen der vorliegenden Erfindung einsetzbar, beispielsweise die oben genannten Testelemente. Dementsprechend kann das Messgerät beispielsweise mindestens eine Applikationsöffnung aufweisen, welche eingerichtet ist, um eine Aufgabe einer Probe der Körperflüssigkeit auf das Testelement zu ermöglichen. Beispielsweise kann diese Applikationsöffnung derart eingerichtet sein, dass von außen ein Testelement in die Applikationsöffnung eingeführt wird, beispielsweise ein optisches und/oder elektrochemisches Testelement. Alternativ oder zusätzlich kann durch die Applikationsöffnung auch ein im Inneren des Messgeräts aufgenommenes Testelement, beispielsweise aus einem Testelement-Magazin, ausgefahren werden, so dass eine Probcnaufgabenstelle des Testelements für einen Benutzer zugänglich wird. Wiederum alternativ oder zusätzlich kann die Applikationsöffnung auch derart eingerichtet sein, dass durch diese Applikationsöffnung eine Applikationsstelle auf einem Testelement vorübergehend freigelegt wird, so dass ein Benutzer eine entsprechende flüssige Probe, beispielsweise einen Blutstropfen, aufbringen kann. Verschiedene weitere Ausgestaltungen sind denkbar. Vorzugsweise ist das Messsystem derart ausgestaltet, dass die Aufgabe der Probe bei in der Aufnahme aufgenommenem Messgerät durchführbar ist. Dies kann beispielsweise dadurch gewährleistet sein, dass das Spielzeug, wie oben dargestellt, mindestens eine entsprechende Öffnung aufweist, welche vorzugsweise vollständig unbedeckt ist und/oder welche durch einen entsprechenden Öffnungsmechanismus freigegebbar und welche die Applikationsöffnung für einen Benutzer auch bei in der Aufnahme aufgenommenem Messgerät möglich macht.

Wie oben bereits beschrieben, ist es besonders bevorzugt, wenn das Spielzeug zumindest teilweise als Figur ausgestaltet ist, also als Wesen, welchem - zumindest in der Fantasie eines Kindes - ein eigener Charakter zugewiesen werden kann. Dementsprechend kann das Spielzeug beispielsweise zumindest teilweise als menschliche Figur, als tierische Figur oder als Fantasiefigur ausgestaltet sein. In diesem Fall ist es besonders bevorzugt, wenn die Aufnahme eine Öffnung aufweist, wobei die Öffnung zumindest teilweise in einem Gesichtsbereich der Figur angeordnet ist. Beispielsweise kann die Öffnung einen vollständigen Bereich eines Gesichts der Figur umfassen und/oder lediglich einen Teilbereich des Gesichts, beispielsweise einen Bereich der Augen und/oder des Mundes. In diesem Fall ist es bevorzugt, wenn die Öffnung derart ausgestaltet ist, dass das Anzeigenelement bei in der Aufnahme aufgenommenem Messgerät zumindest teilweise in der Öffnung erkennbar ist. Dadurch ist es beispielsweise möglich, dass mittels eines Displays des Anzeigenelements Teile des Gesichts der Figur dargestellt werden. Auf diese Weise kann das Anzeigenelement die übrigen Erscheinungsmerkmale des Spielzeugs, insbesondere der Figur, ergänzen. Da das Anzeigenelement vorzugsweise als variables Anzeigenelement ausgestaltet ist, lassen sich auf diese Weise beispielsweise Augen und/oder sonstige Bestandteile des Gesichts darstellen, welche auch veränderbar ausgestaltet sein können.

Das Messgerät kann eine Steuerung aufweisen, welche beispielsweise ganz oder teilweise Bestandteil einer zentralen Steuerung des Messgeräts sein kann, welche jedoch auch als unabhängige, dezentrale Steuerung ausgestaltet sein kann. Diese zentrale Steuerung kann beispielsweise die oben beschriebenen Messaufgaben zum Nachweis des mindestens einen Analyten ganz oder teilweise steuern und/oder kontrollieren. Beispielsweise können die zentrale Steuerung und/oder die Steuerung ein oder mehrere Datenverarbeitungsgeräte umfassen, insbesondere einen oder mehrere Mikrocomputer. Weiterhin können ein oder mehrere Speicherelemente umfasst sein.

Die Steuerung kann eingerichtet sein, um in zumindest einem Betriebsmodus zumindest Teile eines Gesichts der Figur auf dem Anzeigenelement darzustellen. Dabei kann die Steuerung insbesondere derart ausgestaltet sein, dass ein Gesichtsausdruck des Gesichts entsprechend einer oder mehrerer Bedingungen des Messgeräts veränderlich ist. So kann ein Gesichtsausdruck beispielsweise eine Ausgestaltung von Augen und/oder eines Mundes und/oder übriger Gesichtsbestandteile umfassen. Der Gesichtsausdruck kann beispielsweise entsprechend einer oder mehrerer der folgenden Bedingungen verändert werden: entsprechend eines Messergebnisses des Nachweises des Analyten in der Körperflüssigkeit; entsprechend eines Zeitpunktes, insbesondere eines Zeitpunktes einer durchzuführenden Messung. Alternativ oder zusätzlich ist jedoch auch eine Anpassung auf weitere Bedingungen des Messgeräts möglich. Auf diese Weise kann ein kindlicher Benutzer beispielsweise auf kindgerechte Weise über die Messergebnisse und deren Bedeutung informiert werden, beispielsweise durch eine einfache "gut"-"schlecht"-Aussage. Auch eine kindgerechte, freundliche Erinnerung an eine nunmehr durchzuführende Messung kann auf diese Weise erfolgen.

Wie oben dargestellt, ist das Anzeigenelement vorzugsweise ganz oder teilweise als optisches Anzeigenelement ausgestaltet. Das Messgerät, insbesondere das Anzeigenelement, kann jedoch weiterhin auch weitere Elemente aufweisen, welche eine Interaktion mit einem Benutzer ermöglichen, beispielsweise Elemente, welche vibrieren, akustische Signale ausgeben oder thermische Signale ausgeben. Auch eine Kombination verschiedener derartiger oder weiterer Interaktionselemente ist denkbar. Besonders bevorzugt ist es, wenn das Messgerät, insbesondere das Anzeigenelement, weiterhin mindestens ein akustisches Element aufweist, wobei das akustische Element eingerichtet ist, um eine Ausgabe akustischer Signale, insbesondere eine Sprachausgabe, vorzunehmen. Eine derartige Ausgabe akustischer Signale, insbesondere die Sprachausgabe, kann beispielsweise den Charakter der dargestellten Figur des Messsystems weiter unterstützen bzw. auf den jeweiligen Charakter eingestellt sein. So kann beispielsweise eine Stimme, eine Wortwahl, eine Sprache oder Ähnliches auf den jeweiligen Charakter und/oder die jeweilige Zielgruppe angepasst werden, beispielsweise wiederum eine Altersstufe, eine landesspezifische Zielgruppe, eine geschlechtsspezifische Zielgruppe oder Ähnliches. Die Steuerung kann beispielsweise einen Speicher umfassen, in welchem mehrere Modi mit unterschiedlichen Sprachelementen gespeichert sind, welche beispielsweise entsprechend der Zielgruppe und/oder beispielsweise entsprechend der dargestellten Figur ausgewählt werden können. Auch die Sprachausgabe kann eine oder mehrere der oben genannten Funktionen umfassen. Insbesondere kann mittels der Sprachausgabe eine Ausgabe mindestens eines Messergebnisses des Nachweises des Analyten in der Körperflüssigkeit erfolgen. Alternativ oder zusätzlich kann auch eine Stellungnahme zu mindestens einem Messergebnis des Nachweises des Analyten in der Körperflüssigkeit abgegeben werden. Wiederum kann, ebenfalls alternativ oder zusätzlich, auch eine Aufforderung zur Vornahme einer Messung erfolgen und/oder eine Aufforderung zur Vornahme mindestens einer sonstigen Handlung, insbesondere zum Austausch eines Energiespeichers. Auch eine Antwort auf einen Zuruf eines Benutzers kann ausgegeben werden.

Alternativ oder zusätzlich zu dem mindestens einen akustischen Element, welches akustische Signale ausgeben kann, kann das Messgerät weiterhin auch mindestens ein Eingabeelement zur Eingabe akustischer Signale umfassen. Beispielsweise kann dieses Eingabeelement ein oder mehrere Mikrofone umfassen. Weitere Bausteine können vorgesehen sein, beispielsweise Spracherkennungsmodule, welche beispielsweise auch ganz oder teilweise in der Steuerung durch Softwarebausteine implementiert sein können. Dieses Eingabeelement zur Eingabe akustischer Signale kann beispielsweise zur Eingabe von Sprachbefehlen eingerichtet sein. Diese mögliche Ausgestaltung kann den Eindruck für einen kindlichen Benutzer steigern, dass das Messsystem mit einem eigenen Charakter ausgestattet ist, und kann die Interaktion eines kindlichen Patienten mit dem Messsystem verbessern. Alternativ oder zusätzlich zu einer Kommunikation über Tasten und ähnliche Elemente kann eine natürlichere Kommunikation erfolgen, was die Benutzerfreundlichkeit weiter steigern kann. Zudem kann durch die Ausgestaltung des Messsystems als Figur und die genannten optionalen Ausgestaltungen, welche den Charakter der Figur unterstützen, die Hemmschwelle für einen kindlichen Benutzer zur Benutzung des Messsystems weiter gesenkt werden. So kann dem kindlichen Benutzer insbesondere die Angst vor einer Messung weitgehend genommen werden. Weiterhin kann, beispielsweise durch einen Suchruf des Benutzers und eine entsprechende Antwort des Messsystems auch ein Auffinden des Messsystems erleichtert werden. Auch dies unterstützt die Kinderfreundlichkeit des Messsystems, da erfahrungsgemäß Benutzer im Kindesalter zu Unordnung neigen und die Gefahr besteht, dass ein Messsystem in Form eines Spielzeugs in allgemeiner Unordnung eines Spielzimmers unauffindbar ist.

Wie oben dargestellt, kann das Messgerät insbesondere auch mindestens ein Speicherelement aufweisen, insbesondere einen flüchtigen und/oder nicht flüchtigen Speicher. Dabei ist es besonders bevorzugt, wenn in dem Speicherelement eine Mehrzahl von Sätzen von Charakterparametern unterschiedlicher Arten von Spielzeugen hinterlegt ist. Beispielsweise können dies Charakterparameter unterschiedlicher Arten von Figuren sein. Unter Charakterparametern ist dabei allgemein ein Satz von Parametern zu verstehen, welche speziell auf eine bestimmte Figur oder eine sonstige Erscheinungsform des Spielzeugs angepasst sind. Beispielsweise können dies Stimmen einer akustischen Ausgabe, Gesichtsausdrücke, Sprachen oder Ähnliches sein. Entsprechend des jeweiligen, speziell verwendeten Spielzeugs kann dann für das Messsystem ein bestimmter Satz an Charakterparametern auswählbar sein. Diese Auswahl kann beispielsweise durch ein Menü ermöglicht werden, beispielsweise durch ein Elternteil oder durch den kindlichen Benutzer selbst. Auf diese Weise kann beispielsweise ein Elternteil, wenn das Spielzeug gewechselt wird, um einem anderen Kind die Benutzung des Messsystems zu ermöglichen, einen neuen, auf dieses spezielle Spielzeug angepassten Satz von Charakterparametern auswählen. Alternativ oder zusätzlich kann die Auswahl jedoch auch durch einen Hersteller erfolgen, beispielsweise im Rahmen des oben beschriebenen Herstellungsverfahrens und/oder im Rahmen der Verwendung des oben beschriebenen Kits.

Das Spielzeug selbst kann auf verschiedene Weisen ausgestaltet sein und weist, wie oben dargestellt, vorzugsweise eine Spielzeughülle auf, welche die äußere Gestalt des Spielzeugs bestimmt. Das Spielzeug kann dabei als rein passives Spielzeug ausgestaltet sein, ohne eigene Funktionen, wie beispielsweise Bewegungsfunktionen und/oder Ausgaben akustischer und/oder optischer Signale. Alternativ kann das Spielzeug jedoch auch selbst mit mindestens einer Spielzeugsteuerung ausgestattet sein, welche beispielsweise wiederum ein oder mehrere Datenverarbeitungsgeräte umfassen kann und welche eingerichtet sein kann, um mindestens eine spielzeugspezifische Funktion durchzuführen. Beispielsweise kann diese spielzeugspezifische Funktion eine Bewegungsfunktion des Spielzeugs und/oder die Ausgabe akustischer und/oder optischer Signale sein. Alternativ oder zusätzlich sind jedoch auch andere Arten von Spielzeugfunktionen steuerbar. Diese Spielzeugsteuerung, welche vorzugsweise unabhängig von dem Messgerät ausgestaltet und einsetzbar sein soll, kann vorzugsweise mindestens eine Schnittstelle aufweisen. Beispielsweise kann diese Schnittstelle eine drahtgebundene und/oder drahtlose Schnittstelle sein. Diese Schnittstelle kann zum Austausch von Daten und/oder Steuerbefehlen mit dem Messgerät ausgestaltet sein, wobei die Spielzeugsteuerung eingerichtet sein kann, um mit einer Steuerung des Messgeräts zu kommunizieren. Auf diese Weise können beispielsweise Informationen über die Art des Spielzeugs an das Messgerät übermittelt werden. So kann das Messgerät beispielsweise eingerichtet sein, um, entsprechend der Kommunikation mit der Spielzeugsteuerung, mindestens eine Funktion an das jeweilige verwendete Spielzeug anzupassen. Beispielsweise kann dies, wie oben dargestellt, die Auswahl eines oder mehrerer Sätze von Charakterparametern sein, welche speziell auf die gewählte Art des Spielzeugs angepasst sein können. Alternativ oder zusätzlich kann jedoch auch beispielsweise eine Bewegung des Spielzeugs und/oder einer Ausgabe akustischer und/oder optischer Signale zwischen dem Spielzeug und dem Messgerät koordiniert werden.

In Verallgemeinerung dieses Gedankens kann das Spielzeug mindestens einen Identifikator aufweisen, welcher mindestens eine Information über das Spielzeug aufweist. Dieser mindestens eine Identifikator kann dabei auf unterschiedliche Weisen ausgestaltet sein, vorzugsweise als elektronischer Identifikator, insbesondere als berührungslos auslesbarer elektronischer Identifikator. Beispielsweise kann das Spielzeug zu diesem Zweck einen Hochfrequenz-Chip (RFID-Chip) aufweisen, welcher berührungslos elektronisch auslesbar ist, beispielsweise mittels eines entsprechenden Lesegeräts. Derartige Lesegeräte sind bereits heute in vielen Messgeräten enthalten, so dass eine Änderung der Hardware üblicher Messgeräte in vielen Fällen nicht erforderlich ist. Auch andere Arten von Identifikatoren sind grundsätzlich möglich. Der mindestens eine Identifikator kann unabhängig von der optionalen Spielzeugsteuerung ausgestaltet sein, kann jedoch auch ganz oder teilweise in die Spielzeugsteuerung integriert sein.

Die mindestens eine Information über das Spielzeug kann beispielsweise mindestens eine Information über die Art des jeweiligen Spielzeugs umfassen, beispielsweise eine Information über eine durch das Spielzeug verkörperte Figur. Das Messgerät kann eingerichtet sein, um den Identifikator auszulesen, beispielsweise durch ein entsprechendes Lesegerät. Dieses Auslesen kann vorzugsweise automatisch erfolgen, beispielsweise beim Einlegen des Messgeräts in die Aufnahme des Spielzeugs. Diese Aufnahme kann auch eine Schnittstelle zur Kommunikation mit dem Messgerät umfassen, beispielsweise eine drahtlose und/oder drahtgebundene Schnittstelle. Auf diese Weisen können Informationen, unter anderem beispielsweise die in dem Identifikator enthaltenen Informationen, an das Messgerät übermittelt werden. Das Messgerät kann dann wiederum eingerichtet sein, um beispielsweise mindestens eine Funktion an die Information anzupassen. Wiederum kann diese Anpassung beispielsweise die Auswahl und/oder Anpassung eines Satzes von Charakterparametern umfassen. Auf diese Weise kann ein Wechsel des Spielzeugs automatisch an das Messgerät übermittelt werden, so dass keine zusätzliche manuelle Anpassung des Messgeräts erforderlich ist.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: eine schematische Darstellung eines erfindungsgemäßen Messsystems und eines Messgeräts;
- Figur 2: eine teilweise Darstellung des Messsystems und des Messge- räts gemäß Figur 1;
- Figur 3: eine symbolische Darstellung der Verwendung des Messsys- tems für verschiedene Altersstufen von Kindern;
- Figuren 4A und 4B: eine Vorder- und eine Seitenansicht eines im Rahmen des Messsystems einsetzbaren Messgeräts;
- Figur 5: eine schematische Darstellung einer Aufnahme in einem Spielzeug für ein erfindungsgemäßes Messsystem;
- Figuren 6A und 6B: eine schematische Darstellung der Aufnahme des Messgeräts in einem Spielzeug gemäß Figur 5;
- Figur 7: ein Ausführungsbeispiel einer Ruf- und Antwortinteraktion zwischen Benutzer und Messsystem;
- Figur 8: eine schematische Darstellung von möglichen visuellen und akustischen Anzeigen des Messsystems;
- Figur 9: ein Beispiel einer Eingabe eines Teststreifens in eine Einga- beöffnung eines Messgeräts;
- Figuren 10A und 10B: verschiedene Ansichten der Verwendung des Messgeräts, unabhängig vom Messsystem;
- Figur 11: eine perspektivische Darstellung des Messsystems; und
- Figur 12: ein Ausführungsbeispiel eines Kits für ein erfindungsgemä- ßes Messsystem.

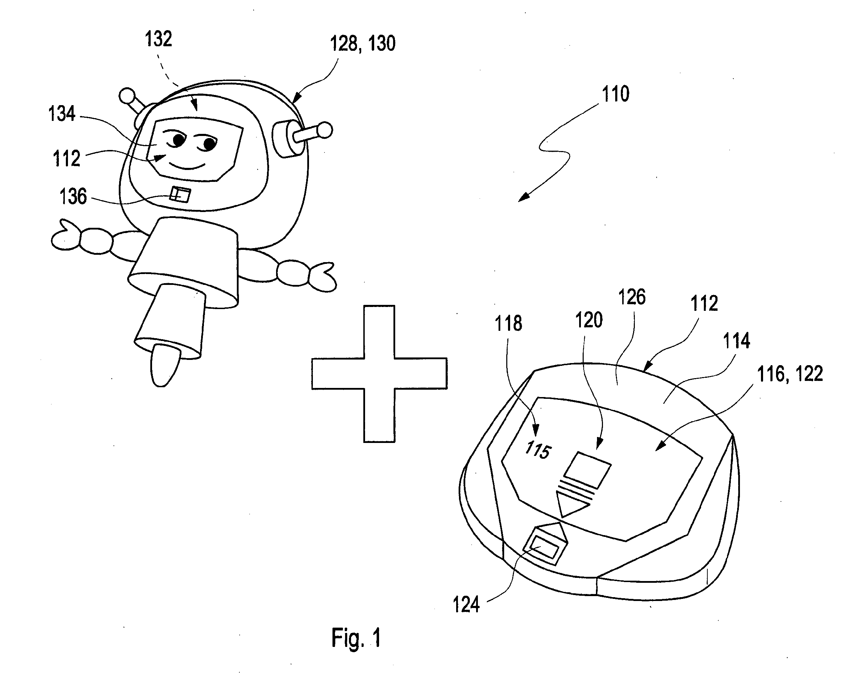

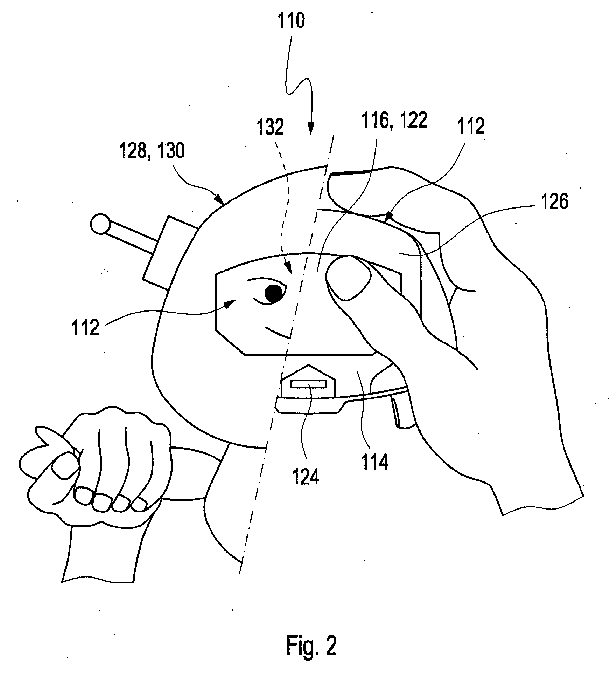

In den Figuren 1 und 2 ist in verschiedenen Darstellungen ein erfindungsgemäßes Messsystem 110 zum Nachweis eines Analyten in einer Körperflüssigkeit gezeigt. Im Folgenden sei, ohne Beschränkung möglicher weiterer Ausgestaltungen, angenommen, dass das Messsystem zur Bestimmung einer Blutglucosekonzentration eingerichtet ist.

Zu diesem Zweck umfasst das Messsystem 110 ein Messgerät 112, welches in der Darstellung gemäß Figur 1 rechts unten separat dargestellt ist. Dieses Messgerät 112 weist ein Gehäuse 114 auf, welches beispielsweise ganz oder teilweise aus starrem Kunststoff hergestellt sein kann. Auch eine mehrteilige Ausgestaltung dieses Gehäuses 114 ist möglich, beispielsweise eine Ausgestaltung mit unterschiedlichen Kunststoffen, beispielsweise um eine Ergonomie des Messgeräts 112 zu verbessern. Das Gehäuse 114 stellt beispielsweise einen Schutz des Innenraums des Messgeräts 112 gegenüber Feuchtigkeit und/oder Verschmutzungen bereit sowie einen Schutz gegenüber mechanischen Beschädigungen. Das Gehäuse 114 ermöglicht, dass das Messgerät 112 auch unabhängig von dem beschriebenen Messsystem 110 einsetzbar ist. Beispielsweise kann das Messgerät 112 einem handelsüblichen herkömmlichen Messgerät 112 entsprechen.

Das Messgerät 112 weist ein Anzeigenelement 116 auf. Dieses Anzeigenelement 116 ist in dem dargestellten Ausführungsbeispiel als optisches Anzeigenelement ausgestaltet und umfasst beispielsweise ein Display. Hier lassen sich verschiedene Arten von Displays einsetzen, beispielsweise Flüssigkristallanzeigen und/oder organische Leuchtdioden-Displays. Mittels des Anzeigenelements 116 können beispielsweise Messwerte dargestellt werden. Diese Messwerte sind in Figur 1 symbolisch mit der Bezugsziffer 118 bezeichnet. Auch andere Darstellungen sind jedoch grundsätzlich möglich. Weiterhin können auf dem Anzeigenelement beispielsweise auch Benutzungsanweisungen dargestellt werden, welche in Figur 1 symbolisch mit der Bezugsziffer 120 bezeichnet sind, und welche beispielsweise zur Eingabe eines (in Figur 1 nicht dargestellten) Testelements auffordern können.

Weiterhin weist das Messgerät 118 zusätzlich ein oder mehrere Bedienelemente auf. Diese Bedienelemente können unabhängig von dem Anzeigenelement 116 ausgestaltet sein, können jedoch auch ganz oder teilweise in das Anzeigenelement 116 integriert sein. Beispielsweise kann das Anzeigenelement 116 ganz oder teilweise als Touch-Screen ausgestaltet sein, was in Figur 1 symbolisch durch die Bezugsziffer 122 angedeutet ist. Auf diese Weise lässt sich beispielsweise eine Menüsteuerung oder eine andere Art der Eingabe von Steuerbefehlen und/oder Parametern in das Messgerät 112 durch einen Benutzer realisieren.

Weiterhin umfasst das Messgerät 112 in dem dargestellten Ausführungsbeispiel eine Applikationsöffnung 124. Diese Applikationsöffnung 124 ist in dem dargestellten Ausführungsbeispiel unterhalb des Anzeigenelements 116 auf einer Frontseite 126 angeordnet. Die Anordnung der Applikationsöffnung 124 auf der Frontseite 126, also auf derselben Seite, auf welcher auch das Anzeigenelement 116 und/oder eines von mehreren Anzeigenelementen 116 angeordnet ist, erleichtert bei der Integration in das Messsystem 110 den gleichzeitigen Zugang zum Anzeigenelement 116 und der Applikationsöffnung 124. Auch andere Ausgestaltungen sind jedoch grundsätzlich möglich.

Durch die Applikationsöffnung 124 wird beispielsweise ein Testelement, insbesondere ein Teststreifen, in das Messgerät 112 zumindest teilweise eingegeben und/oder aus dem Messgerät 112 zumindest teilweise ausgegeben. Üblicherweise weist ein derartiges Testelement eine Applikationsstelle auf, auf welcher die flüssige Probe aufgebracht wird. Das Eingeben bzw. Ausgeben des Testelements in die Applikationsöffnung bzw. aus der Applikationsöffnung 124 erfolgt dabei in der Regel derart, dass diese Applikationsstelle freiliegt, so dass die Applikation der flüssigen Probe möglich ist.

Bei dem Messgerät 112 kann es sich um ein handelsübliches Messgerät handeln, so dass für weitere mögliche Ausgestaltungen dieses Messgeräts insbesondere auf den Stand der Technik verwiesen werden kann. Die dargestellte Ausführungsform ist jedoch eine bevorzugte Ausführungsform im Rahmen des erfindungsgemäßen Messsystems 110.

Das Messgerät 112 ist in Figur 1 rechts unten sowie in Figur 2 im rechten Teilbild dargestellt. Weiterhin umfasst das Messsystem 110, wie in Figur 1 links oben und in Figur 2 im linken Teilbild dargestellt, ein Spielzeug 128. Bei diesem Spielzeug 128 kann es sich beispielsweise um ein modifiziertes Stofftier oder, wie in den Figuren 1 und 2 symbolisch dargestellt, um eine Figur in Form einer Puppe oder einer Fantasiefigur handeln.

Das Spielzeug 128 umfasst eine Spielzeughülle 130, welche die äußere Gestalt des Spielzeugs 128 im Wesentlichen bestimmt. Diese Spielzeughülle 130 kann, je nach Spielzeug 128, beispielsweise aus einem harten und/oder verformbaren, insbesondere flexiblen Material hergestellt sein. Hierfür lassen sich natürliche Materialien und/oder künstliche Materialien einsetzen. Insbesondere lassen sich Material, Farbe, Form, dargestellte Figur oder sonstige Erscheinungsformen der Spielzeughülle 130 an eine bestimmte Zielgruppe anpassen, wie unten näher ausgeführt wird.

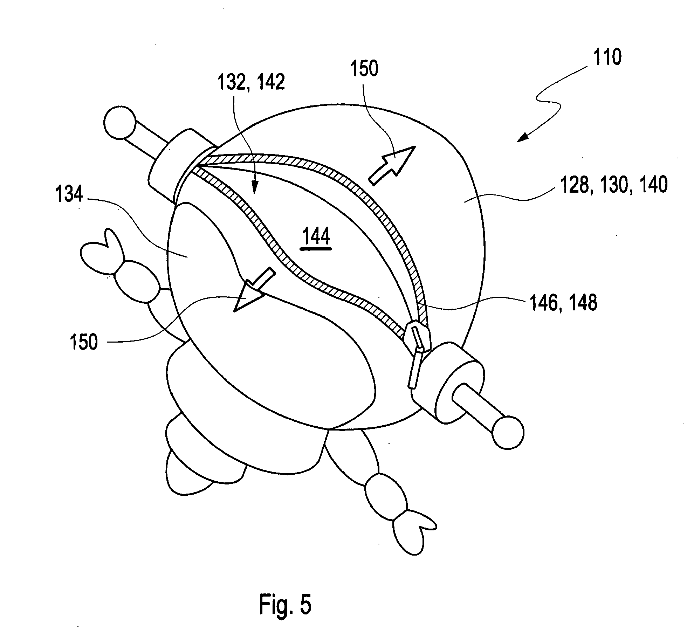

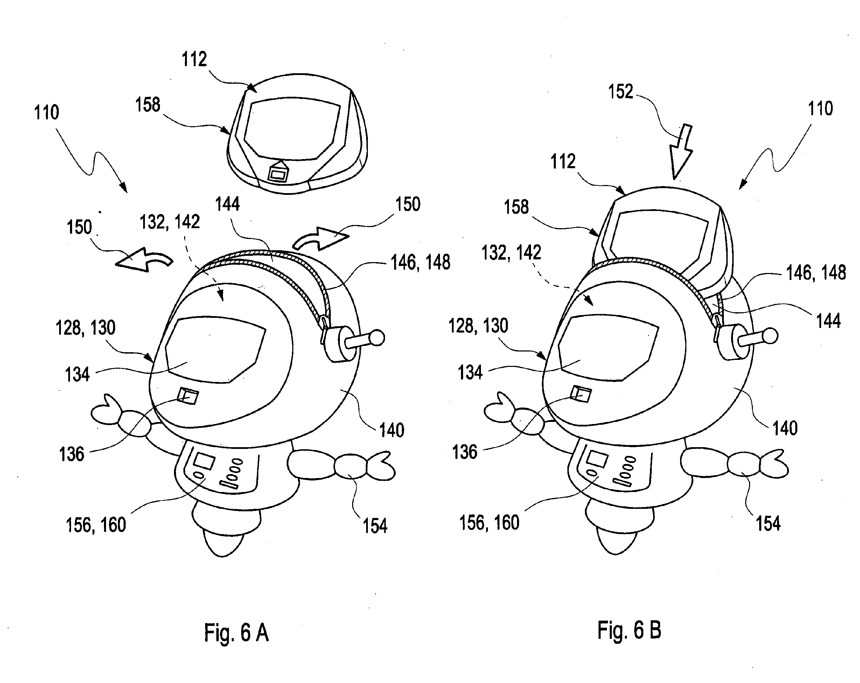

Das Spielzeug 128 weist eine Aufnahme 132 auf, welche in den Figuren 1 und 2 lediglich angedeutet ist und welche sich ganz oder teilweise im Inneren der Spielzeughülle 130 befindet. Diese Aufnahme 132 wird unten, beispielsweise anhand der Figuren 5 und 6A und 6B in weiteren möglichen Einzelheiten näher erläutert. Die Aufnahme 132 ist derart ausgestaltet, dass bei in der Aufnahme 132 aufgenommenem Messgerät 112 das Anzeigenelement 116 des Messgeräts 112 zumindest teilweise zugänglich ist. Zu diesem Zweck kann die Spielzeughülle 130 im Bereich der Aufnahme 132 eine oder mehrere Öffnungen 134, 136 aufweisen, wie in Figur 1 angedeutet. Eine erste Öffnung ist 134 vorgesehen, welche beispielsweise im Wesentlichen der Fläche des Anzeigenelements 116 entspricht. Die Öffnung kann dabei vollständig unverschlossen sein oder, was ebenfalls noch unter den Begriff einer Öffnung zu subsumieren sein soll, durch eine - vorzugsweise transparente - Abdeckung ganz oder teilweise verschlossen sein, so dass die mittels des Anzeigenelements 116 bereitgestellten optischen Informationen nach wie vor durch einen Benutzer wahrnehmbar sein können. Die zweite Öffnung 136 kann beispielsweise im Bereich der Applikationsöffnung 124 des Messgeräts 112 angeordnet sein, so dass durch diese Öffnung 136 eine Eingabe und/oder Ausgabe eines oder mehrerer Testelemente möglich ist. Alternativ oder zusätzlich kann diese zweite Öffnung 136 jedoch auch ganz oder teilweise mit der ersten Öffnung 134 kombiniert werden. Diese zweite Öffnung 136 ist vorzugsweise ganz oder teilweise unbedeckt und/oder mit einem Öffnungsmechanismus zumindest freigebbar, so dass die Eingabe und/oder Ausgabe des Testelements erfolgen kann.

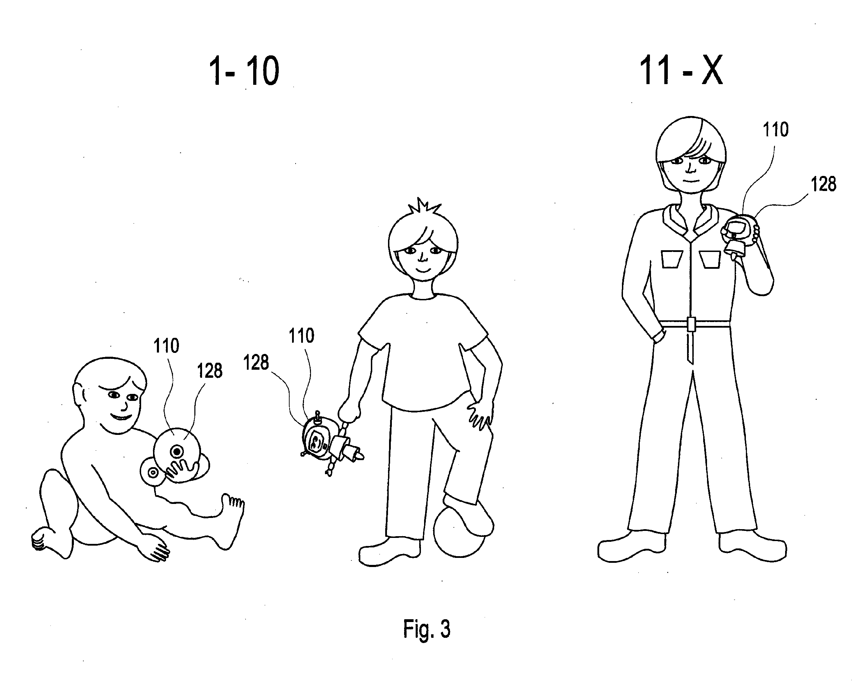

In Figur 3 ist die Anpassung des Messsystems 110 auf Zielgruppen kindlicher Benutzer symbolisch dargestellt. Insbesondere zeigt diese Darstellung, wie das äußere Erscheinungsbild des Messsystems 110 erfindungsgemäß auf die unterschiedlichen Altersgruppen der Benutzer angepasst werden kann. Während Benutzer im Kleinkindalter von 1 bis 3 Jahren in der Regel einfache Spielzeuge 128 bevorzugen, welche vorzugsweise aus weichem Material hergestellt sind und in der Regel in farblichen Pastelltönen gehalten sein können, bevorzugen Benutzer im Alter von ca. 4 bis 10 Jahren Spielzeuge 128 mit Figurendarstellung mit ausgeprägteren charakterlichen Details. Hier kommen beispielsweise bestimmte Puppen, aus Kinofilmen, Funk und Fernsehen bekannte Figuren oder Ähnliches in Betracht. Teenager hingegen, ab dem Alter von ca. 11 Jahren, bevorzugen hingegen, wie in Figur 3 ebenfalls angedeutet (rechtes Teilbild) komplexere Spielzeuge 128, welche auch beispielsweise wiederum von einer Figurendarstellung abweichen können oder welche ebenfalls an bekannte Figuren aus Kino, Funk und Fernsehen angepasst sein können. Sämtliche Messsysteme 110 in Figur 3 können dabei auf demselben Messgerät 112 basieren, wobei lediglich die Spielzeughülle 130 bzw. das Spielzeug 128 ausgetauscht werden muss. So kann beispielsweise auch eine Familie mit einem einzigen Messgerät 112 auskommen, zu welchem lediglich Spielzeuge 128 ausgetauscht werden müssen, sofern kindliche Benutzer das Messsystem 110 einsetzen sollen.

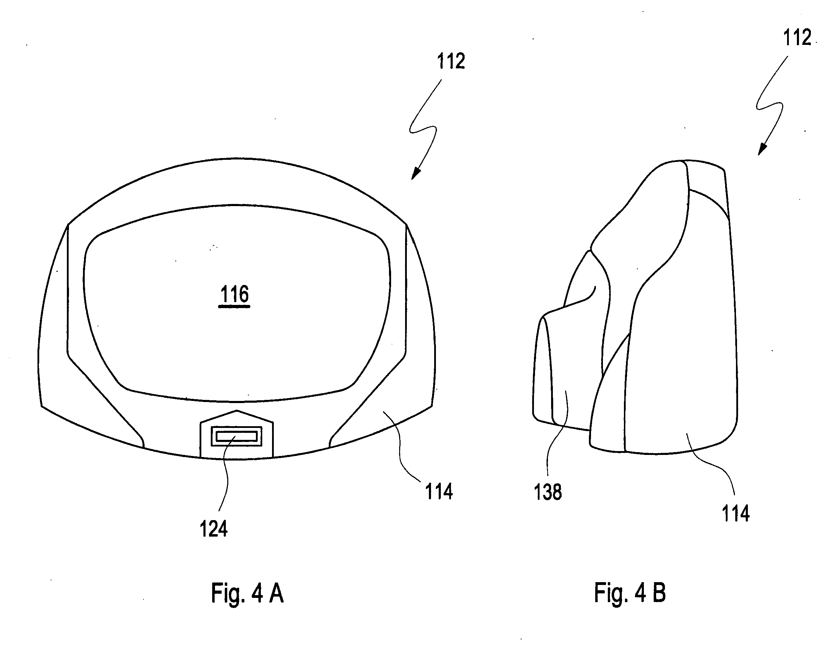

In den Figuren 4A und 4B sind eine Vorderansicht (Figur 4A) bzw. eine Seitenansicht (Figur 4B) einer alternativen Ausführungsform eines Messgeräts 112 dargestellt, welches im Rahmen des erfindungsgemäßen Messsystems 110 eingesetzt werden kann. Prinzipiell ist dieses Messgerät 112 ähnlich zu dem in Figur 1, Teilbild rechts unten, dargestellten Messgerät 112 ausgestaltet und umfasst wiederum ein Gehäuse 114, welches ebenfalls ähnlich zu dem in Figur 1 dargestellten Gehäuse 114 ausgestaltet ist. Weiterhin sind wiederum ein Anzeigenelement 116 sowie eine Applikationsöffnung 124 umfasst. Insbesondere die Seitendarstellung gemäß Figur 4B zeigt, dass das Gehäuse 114 durch entsprechende Griffelemente 138 ergonomisch ausgestaltet sein kann, um eine Handhabung durch einen Benutzer auch außerhalb des Messsystems 110 problemlos zu ermöglichen. Hierdurch wird bewirkt, dass das Messgerät 112 auch für Benutzer, welche das Messgerät 112 ohne Spielzeug 128 einsetzen möchten, attraktiv ausgestaltet ist.

Anhand der Figuren 5 und 6A und 6B soll ein Ausführungsbeispiel eines möglichen Messsystems 110, eines für das Messsystem 110 einsetzbaren Spielzeugs 128 sowie eines Verfahrens zur Herstellung des Messsystems 110 erläutert werden. Bei diesem Ausführungsbeispiel weist, wie in der perspektivischen Darstellung schräg von oben gemäß Figur 5 erkennbar, das Spielzeug 128 wiederum eine Spielzeughülle 130 auf, welche hier beispielsweise wiederum als Fantasiefigur ausgestaltet ist. Auch andere Ausgestaltungen sind jedoch denkbar.

Die Figur weist in dem dargestellten Ausführungsbeispiel vorzugsweise einen Kopf 140 auf. In diesem Kopf 140 ist vorzugsweise die oben bereits beschriebene Aufnahme 132 angeordnet. Auch eine andere Anordnung dieser Aufnahme 132 ist jedoch grundsätzlich denkbar. Die Aufnahme 132 umfasst dabei einen Aufnahmeraum 142 im Inneren des Kopfes 140, in welchem das Messgerät 112 zumindest teilweise aufnehmbar ist. Dieser Aufnahmeraum 142 ist durch die genannte Öffnung 134, welche ganz oder teilweise beispielsweise auch mit einem transparenten Material, beispielsweise einer Klarsichthülle, abgedeckt sein kann, versehen, um in diesem Ausführungsbeispiel einen visuellen Zugang zu dem Anzeigenelement 116 zu ermöglichen. Auf diese Weise kann ein zusätzlicher Schutz des Anzeigenelements 116 gewährleistet sein. Alternativ kann die Öffnung 134 jedoch auch vollständig unbedeckt sein, da in der Regel das Gehäuse 114 des Messgeräts 112, welches auch das Anzeigenelement 116 und/oder eine transparente Abdeckung dieses Anzeigenelements 116 umfassen kann, bereits einen ausreichenden Schutz bereitstellen kann.

Die Aufnahme 132 bzw. der Aufnahmeraum 142 weisen vorzugsweise mindestens eine Aufnahmeöffnung 144 auf. Diese mindestens eine Aufnahmeöffnung 144 ist dabei in dem dargestellten Ausführungsbeispiel als Schlitz entlang einer Oberseite des Kopfes 140 ausgestaltet. Vorzugsweise weist diese Aufnahmeöffnung 144 mindestens ein Verschlusselement 146 auf, mittels dessen die Aufnahmeöffnung 144 für den Gebrauch verschließbar und für ein Einfügen und/oder Herausnehmen des Messgeräts 112 zu öffnen bzw. zu schließen ist. Dieses Verschlusselement 146 kann, wie in den Figuren 5, 6A und 6B symbolisch dargestellt, beispielsweise einen Reißverschluss 148 umfassen. Alternativ oder zusätzlich sind jedoch auch andere Arten von Verschlusselementen 146 einsetzbar, beispielsweise Klettverschlüsse, Druckknöpfe, Textilknöpfe oder Ähnliches. Vorzugsweise sollte das Verschlusselement 146 leicht auch durch einen Benutzer zu öffnen sein, wobei jedoch auch beispielsweise eine Kindersicherung vorgesehen sein kann, um ein unerwünschtes Herausnehmen des Messgeräts 112 durch einen kindlichen Patienten zu verhindern.

In den Figuren 6A und 6B ist ein einfaches Herstellungsverfahren zur Herstellung des Messsystems 110 angedeutet. In einem ersten Teilschritt wird in Figur 6A das Verschlusselement 146 geöffnet (angedeutet durch die Pfeile 150) und in einem zweiten, in Figur 6B dargestellten Teilschritt das Messgerät 112 in die Aufnahme 132 eingefügt (angedeutet durch die Bezugsziffer 152).

Das Spielzeug 128 kann als rein passives Spielzeug ausgestaltet sein. Eine derartige Ausgestaltung ist insbesondere bei einer Anwendung durch Kleinkinder von Vorteil. Alternativ kann das Spielzeug 128 jedoch auch über eine komplexere Funktionalität verfügen und mindestens eine spielzeugspezifische Funktion durchführen. Diese Funktion kann beispielsweise eine Kommunikationsfunktion und/oder eine Bewegungsfunktion (beispielsweise in den Figuren 6A und 6B ein Bewegen der Arme 154) oder Ähnliches umfassen. Um diese Spielzeugfunktion aktiv zu steuern, kann das Spielzeug 128 beispielsweise eine Spielzeugsteuerung 156 umfassen, welche in den Figuren 6A und 6B schematisch angedeutet ist. Diese Spielzeugsteuerung 156 kann beispielsweise ein oder mehrere elektronische Bauelemente umfassen, beispielsweise einen oder mehrere Mikroprozessoren. Dabei kann die Spielzeugsteuerung 156 eingerichtet sein, um mit einer Steuerung des Messgeräts 112 über eine oder mehrere in den Figuren nicht dargestellte Schnittstelle zu kommunizieren. Die Steuerung des Messgeräts 112 ist in den Figuren 6A und 6B symbolisch mit der Bezugsziffer 158 bezeichnet. Auch diese Steuerung 158 kann beispielsweise wiederum ein oder mehrere elektronische Bauelemente umfassen, beispielsweise wiederum einen oder mehrere Mikroprozessoren. Wie auch in der Spielzeugsteuerung 156, so kann auch die Steuerung 158 optional ein oder mehrere Speicherelemente umfassen, beispielsweise flüchtige und/oder nicht-flüchtige Speicher. Auf diese Weise können über die Schnittstelle beispielsweise die Steuerung 158 und die Spielzeugsteuerung 156 miteinander Daten und/oder Steuerbefehle austauschen. Auf diese Weise kann die Spielzeugfunktionalität beispielsweise mit der Funktionalität des Messgeräts 112 koordiniert und/oder synchronisiert werden, beispielsweise um entsprechend der Funktionalität des Spielzeugs 128 synchronisiert einen Gesichtsausdruck auf dem Anzeigeelement 116 anzuzeigen.

Weiterhin kann, wahlweise auch unabhängig von der Spielzeugsteuerung 156, das Spielzeug 128 auch einen Identifikator 160 umfassen, welcher in den Figuren 6A und 6B lediglich angedeutet ist und welcher beispielsweise ganz oder teilweise in der Spielzeugsteuerung 156 integriert sein kann oder unabhängig von dieser in dem Spielzeug 128 aufgenommen sein kann. Der Identifikator 160 kann beispielsweise als RFID-Chip ausgestaltet sein. Entsprechend kann das Messgerät 112 eingerichtet sein, um mindestens eine Information aus dem Identifikator 160 auszulesen. Dies kann beispielsweise durch ein entsprechendes Lesegerät erfolgen, welches in den Figuren nicht dargestellt ist. Dieses Lesegerät kann auch ganz oder teilweise mit der Schnittstelle zusammengefasst sein.

Entsprechend der ausgelesenen Information über das Spielzeug 128 kann das Messgerät 112 bzw. die Steuerung 158 eingerichtet sein, um mindestens eine Funktion des Messgeräts an die ausgelesene Information anzupassen. Beispielsweise kann die Information eine Information über eine Art des Spielzeugs 128 umfassen, so dass beispielsweise aus einer Mehrzahl von gespeicherten Parametersätzen von Charakterparametern der korrekte Satz ausgewählt werden kann. Diese Auswahl kann der jeweils eingesetzten Figur des Spielzeugs 128 bzw. der Art des Spielzeugs 128 entsprechen. Die Auslesung und Auswahl kann dabei beispielsweise automatisch erfolgen, so dass keine zusätzliche Benutzereingabe erforderlich ist, wenn gemäß den Figuren 6A und 6B das Messgerät 112 in ein Spielzeug 128 eingefügt wird.

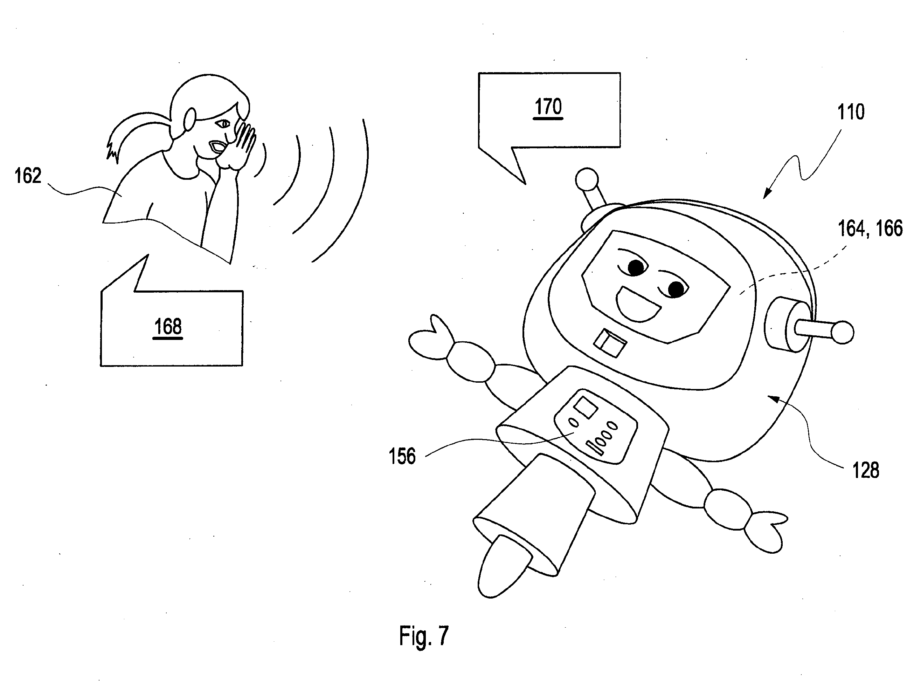

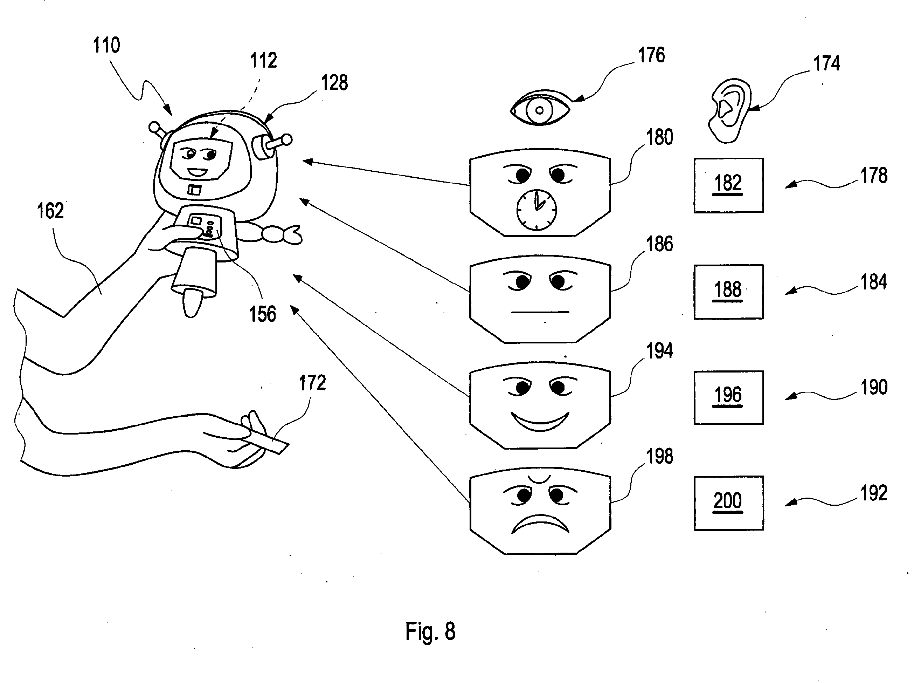

Das Messsystem 110 ist vorzugsweise eingerichtet, um auf verschiedene Weisen mit einem Benutzer zu interagieren. Beispiele einer derartigen Benutzerinteraktion sind in den Figuren 7 und 8 dargestellt. So zeigt Figur 7 ein Ausführungsbeispiel einer Interaktion zwischen einem Messsystem 110 und einem Benutzer 162, beispielsweise einem Patienten oder einer anderen Person. Allgemein wird darauf hingewiesen, dass im Rahmen der vorliegenden Beschreibung der Begriff eines Patienten nicht notwendigerweise einen Krankheitszustand impliziert, so dass der Patient beispielsweise auch eine Person oder ein Tier sein kann, welche beziehungsweise welches lediglich eine Vorsorgeuntersuchung durchführt. Weiterhin wird auch zwischen dem Begriff des Patienten und des Benutzers 162 nicht unterschieden, so dass es sich bei diesen um unterschiedliche Wesen oder identische Wesen handeln kann. Das Messsystem 110 in dem Ausfirhrungsbeispiel gemäß Figur 7 kann beispielsweise ausgestaltet sein wie in den vorhergehenden Ausführungsbeispielen beschrieben, so dass für die mögliche Ausgestaltung dieses Messsystems 110 und des darin aufgenommenen Messgeräts 112 sowie des Spielzeugs 128 beispielsweise auf obige Beschreibung verwiesen werden kann.

Zusätzlich verfügt das Messsystem 110 jedoch, was auch in den vorhergehenden Ausführungsbeispielen optional der Fall sein kann, über ein Eingabesystem 164 für die Eingabe akustischer Signale sowie über ein Ausgabesystem 166, welches ebenfalls für die Ausgabe akustischer Signale eingerichtet sein kann. Dieses Eingabesystem 164 und das Ausgabesystem 166 sind hierbei bei dem Ausführungsbeispiel gemäß Figur 7 symbolisch dem Messgerät 112 zugeordnet. Beispielsweise kann das Messgerät 112 ein oder mehrere Mikrofone als Eingabesystem 164 und/oder ein oder mehrere Lautsprecher als Ausgabesystem 166 aufweisen. Alternativ oder zusätzlich können jedoch auch eines oder beide der Systeme 164, 166 ganz oder teilweise dem Spielzeug 128 zugeordnet sein, beispielsweise der Spielzeugsteuerung 156.

Die Ausgestaltung des Messsystems 110 mit dem Eingabesystem 164 und dem Ausgabesystem 166 ermöglicht beispielsweise einen Suchruf des Benutzers 162, welcher in Figur 7 symbolisch mit der Bezugsziffer 168 bezeichnet ist. So kann dieser Suchruf 168 beispielsweise ein einfaches Ausrufen einer Bezeichnung des Messsystems 110 und/oder des Spielzeugs 128 beinhalten. So kann, was üblicherweise für eine Erhöhung der Akzeptanz bei Benutzern 162 im Kindesalter von Vorteil ist, das Messsystem 110 und/oder das Spielzeug 128 mit einem vom Benutzer und/oder Hersteller vorgegebenen Namen versehen werden, beispielsweise dem Namen "Buddy". So kann der Suchruf 168 beispielsweise ein einfaches Ausrufen des Begriffs "Buddy" und/oder eine komplexere Suchanfrage, beispielsweise "Buddy, wo bist du?" beinhalten.

Das Messsystem 110, insbesondere die Steuerung 158 und/oder die Spielzeugsteuerung 156, können eingerichtet sein, um diesen Suchruf 168 zu beantworten. Diese Antwort ist in Figur 7 symbolisch mit der Bezugsziffer 170 bezeichnet. Wiederum besteht eine Vielzahl von Ausgestaltungsmöglichkeiten für diese Antwort 170, beispielsweise in Form einer Sprachausgabe. Alternativ oder zusätzlich können jedoch auch andere Signale ausgegeben werden, vorzugsweise akustische Signale, wie beispielsweise ein Brummen, ein Signalton oder Ähnliches. Bevorzugt ist jedoch, ebenfalls für eine Erhöhung der Akzeptanz, eine Sprachausgabe, beispielsweise in Form einer Antwort 170 "Hey-hey, ich bin hier!". Die in Figur 7 dargestellte Interaktion mit dem Benutzer 162 ist jedoch lediglich eines von vielen möglichen Ausführungsbeispielen, wobei zahlreiche weitere Ausführungsbeispiele möglich sind.

In Figur 8 ist eine Interaktion mit einem Benutzer 162 symbolisch dargestellt, welche speziell die Messfunktionen des Messgeräts 112 betrifft. Alternativ oder zusätzlich kann diese Interaktion jedoch wiederum auch ganz oder teilweise auch in weiteren Komponenten des Messsystems 110 realisiert sein, insbesondere in Komponenten des Spielzeugs 128, beispielsweise wiederum in der Spielzeugsteuerung 156. Dargestellt ist wiederum ein Messsystem 110, welches wiederum beispielsweise den zuvor beschriebenen Messsystemen 110 entsprechen kann. Auch eine andere Ausgestaltung ist jedoch grundsätzlich möglich. Das Messsystem umfasst in diesem Ausführungsbeispiel ein Messgerät 112, beispielsweise gemäß der obigen Beschreibung, welches beispielsweise auf der Verwendung eines oder mehrerer Testelemente 172 für den qualitativen und/oder quantitativen Nachweis des mindestens einen Analyten in der flüssigen Probe basiert.

In der rechten Bildhälfte der Figur 8 sind dabei verschiedene Signale dargestellt, welche das Messsystem 110 zur Unterstützung der Messfunktion ausgeben kann. Die Interaktion mit dem Benutzer kann dabei, wie in Figur 8 in der rechten Bildhälfte dargestellt, beispielsweise mittels akustischer Signale erfolgen, welche in Figur 8 symbolisch mit der Bezugsziffer 174 bezeichnet sind, und/oder mittels optischer Signale, welche in Figur 8 symbolisch mit der Bezugsziffer 176 bezeichnet sind. Auch eine Kombination dieser Signale ist möglich.

Dabei bezeichnet die Bezugsziffer 178 eine erste Kombination akustischer und optischer Signale 174, 176, welche jedoch auch einzeln realisierbar sind. Bei dieser ersten Kombination 178 erfolgt eine Zeitanzeige 180, in Kombination beispielsweise mit einem akustischen Signal, welches an die Notwendigkeit der Durchführung einer Messung erinnert (Erinnerungssignal 182). Beispielsweise kann dieses Erinnerungssignal 182 wiederum eine Sprachausgabe umfassen, beispielsweise die Sprachausgabe "ok, Zeit zum Messen!".

In einer zweiten Kombination 184 sind akustische und optische Signale 174, 176 zusammengefasst, welche wiederum auch einzeln realisierbar sind. Beispielsweise kann diese zweite Kombination 184 eingesetzt werden, während eine Auswertung der Messung mittels des Testelements 172 erfolgt. Diese zweite Kombination 184 kann beispielsweise eine Warteanzeige 186 als optisches Signal 176 und/oder eine akustische Warteaufforderung 188 umfassen, beispielsweise wiederum eine Sprachausgabe, beispielsweise die Aufforderung "einen Moment noch!".

Weiterhin sind in der rechten Bildhälfte in Figur 8 eine dritte Kombination 190 und eine vierte Kombination 192 dargestellt, welche eine Bewertung von Messergebnissen abgeben. So kann beispielsweise die dritte Kombination 190 eine positive Anzeige 194 als optisches Signal 176 und/oder eine positive akustische Bewertung 196 umfassen. Die positive akustische Bewertung 196 kann beispielsweise wiederum eine Sprachausgabe umfassen, beispielsweise die Sprachausgabe "hey, tolle Werte heute!". Diese dritte Kombination 190 kann somit beispielsweise dann eingesetzt werden, wenn die Messergebnisse innerhalb eines vorgegebenen Sollbereichs liegen. Die vierte Kombination 192 kann dementsprechend eingesetzt werden, um negative Messergebnisse, insbesondere Messergebnisse außerhalb eines Sollbereichs, zu übermitteln und/oder zu bewerten. Diese vierte Kombination 192 kann beispielsweise eine negative Anzeige 198 als optisches Signal 176 umfassen und/oder eine negative akustische Bewertung 200 als akustisches Signal 174, beispielsweise wiederum eine Sprachausgabe, beispielsweise die Sprachausgabe "oh...viel Zucker!".

Auf diese Weise kann, beispielsweise durch die genannten Interaktionen und/oder ähnliche Interaktionen, eine kindgerechte Benutzerführung gewährleistet werden. Die optischen und/oder akustischen Signale 176, 174 können dabei speziell auf das verwendete Spielzeug 128 angepasst sein, beispielsweise indem die optischen Signale 176 einen dem Spielzeug 128 entsprechenden Gesichtsausdruck anpassen. Alternativ oder zusätzlich kann eine Stimme einer Sprachausgabe der akustischen Signale 174 angepasst werden. Zahlreiche weitere Ausführungsformen dieser Interaktion sind möglich, beispielsweise Zwischenwerte zwischen der durch die dritte Kombination 190 dargestellten positiven Situation und der durch die vierte Kombination 192 dargestellten negativen Situation. Insgesamt lässt sich durch diese Interaktion die Akzeptanz des Messsystems 110 erheblich steigern.

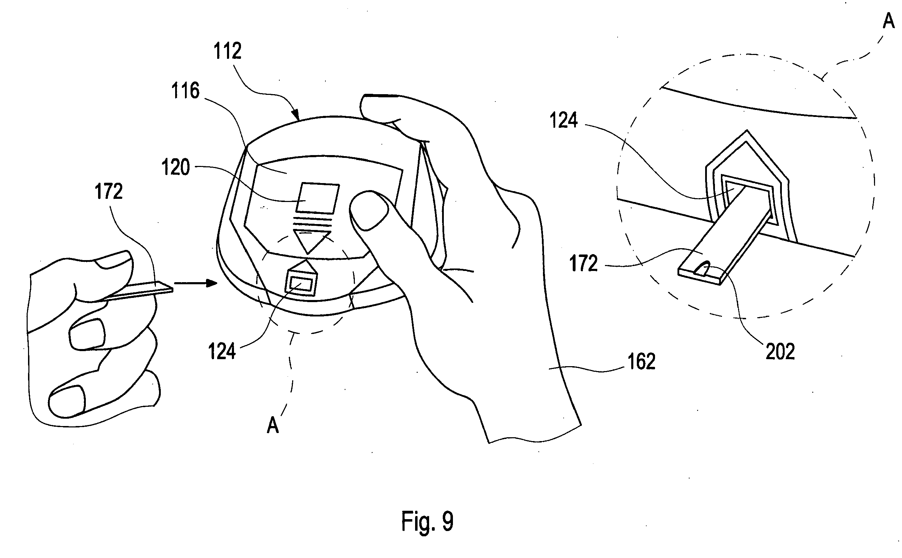

In Figur 9 ist nochmals beispielhaft eine mögliche Benutzung des Messgeräts 112 ohne das Spielzeug 128 dargestellt. Eine Benutzung mit dem Spielzeug 128 kann analog erfolgen. Das Messgerät 112 kann beispielsweise gemäß der obigen Beschreibung ausgestaltet sein. Auch andere Ausgestaltungen sind jedoch grundsätzlich möglich. Das Messgerät 112 umfasst wiederum eine Applikationsöffnung 124, deren Umgebungsbereich im Teilbild rechts oben in Figur 9 (bezeichnet mit dem Buchstaben A) nochmals vergrößert dargestellt ist. Ein Benutzer 162 schiebt ein Testelement 172 in die Applikationsöffnung 124. Dieses Testelement 172, welches beispielsweise als optisches und/oder elektrochemisches Testelement, insbesondere als Teststreifen, ausgestaltet sein kann, kann dann eine Applikationsstelle 202 umfassen, welche in Figur 9 lediglich symbolisch angedeutet ist und auf welche eine flüssige Probe, beispielsweise ein Blutstropfen, aufgebracht werden kann. Nach dem Aufbringen kann das Messgerät 112 beispielsweise automatisch und/oder auf Aufforderung durch den Benutzer 162 einen Nachweis des Analyten in der flüssigen Probe durchführen.

Zahlreiche weitere Möglichkeiten der Interaktion zwischen Messgerät 112 und Testelement 172 sind denkbar. So kann beispielsweise das Testelement 172 auch ganz oder teilweise in dem Messgerät 112 bevorratet sein und durch die Applikationsöffnung 124 für eine Applikation der flüssigen Probe ganz oder teilweise ausgeschoben werden. Wiederum alternativ oder zusätzlich kann die Applikationsöffnung 124 jedoch auch eine einfache Öffnung umfassen, welche einen Testbereich des Testelements 172 für einen Benutzer freigibt, so dass eine Aufgabe der Probe möglich ist. Zahlreiche weitere Ausgestaltungen sind denkbar.

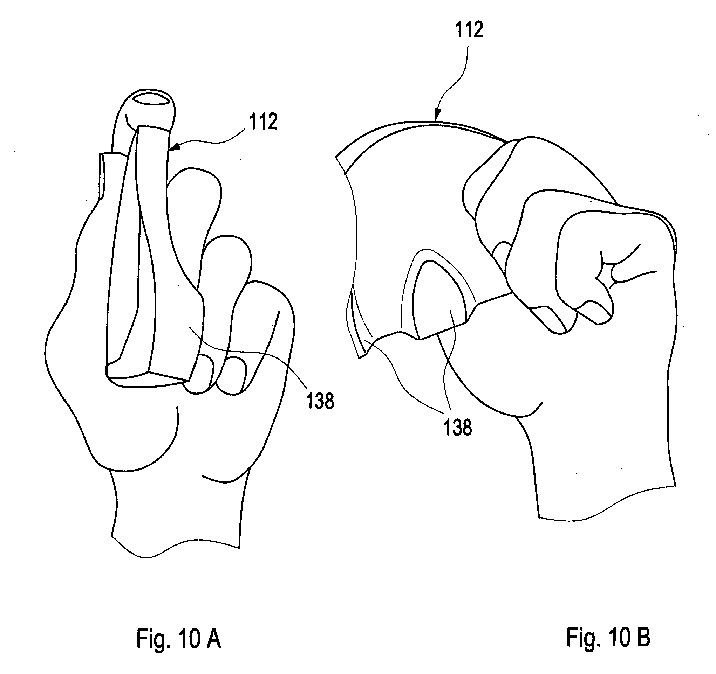

In den Figuren 10A und 10B ist ein weiteres Ausführungsbeispiel eines Messgeräts 112 in einer Seitenansicht (Figur 10A) und einer Rückseitenansicht (Figur 10B) dargestellt, welches im Rahmen des erfindungsgemäßen Messsystems 110 ebenfalls zum Einsatz kommen kann. Das Messgerät 112 unterscheidet sich dabei hinsichtlich der ergonomischen Ausgestaltung leicht von dem Ausführungsbeispiel gemäß den Figuren 4A und 4B, wobei jedoch Ähnlichkeiten bestehen. Wiederum umfasst das Messgerät 112 Griffelemente 138, welche eine Handhabung des Messgeräts 112 erleichtern. Auf diese Weise kann das Messgerät 112 beispielsweise unabhängig von dem Messsystem 110 eingesetzt werden. In Synergiewirkung können diese Griffelemente 138 jedoch auch eingesetzt werden, um eine Halterung des Messgeräts 112 in dem Spielzeug 128 zu stabilisieren.

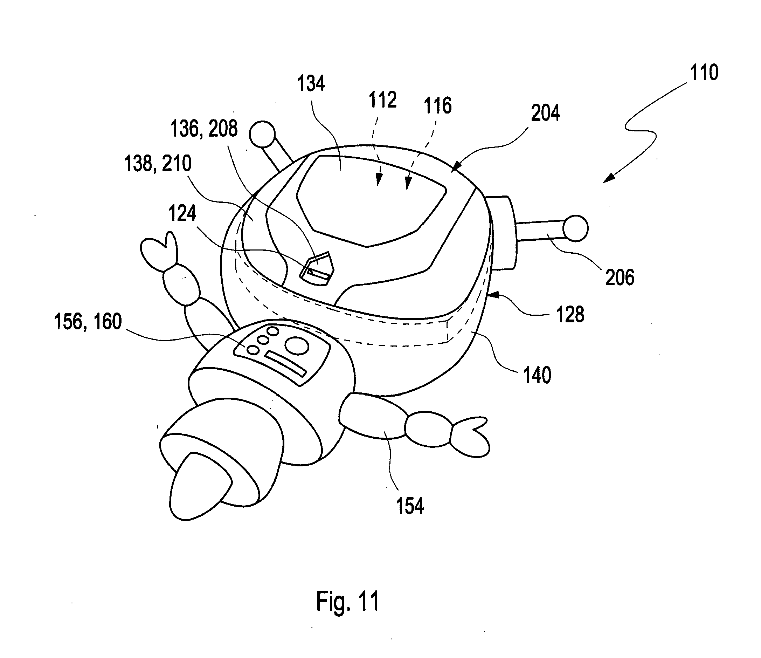

In Figur 11 ist das Messsystem 110 gemäß den zuvor beschriebenen Ausführungsbeispielen nochmals in vergrößerter perspektivischer Darstellung gezeigt. Für Details dieses Ausführungsbeispiels kann beispielsweise auf die Beschreibung der obigen Ausführungsbeispiele verwiesen werden, beispielsweise die Ausführungsbeispiele in den Figuren 1, 2 oder 6A und 6B. Insbesondere ist aus der perspektivischen Darstellung in Figur 11 die Ausgestaltung des Spielzeugs 128 als Figur erkennbar, welche besonders bevorzugt ist. Hierbei ist beispielsweise erkennbar, dass ein Kopf 140 des Spielzeugs 128 derart ausgestaltet sein kann, dass dieser, beispielsweise neben zusätzlichen Elementen des Kopfes, wie zum Beispiel Ohren 206, einen ausgeprägten Gesichtsbereich 204 aufweisen kann. In diesem Gesichtsbereich 204, welcher beispielsweise die Öffnungen 134, 136 umfassen kann, kann die hauptsächliche Zusammenwirkung des Messgeräts 112 mit dem Spielzeug 128 erfolgen, so dass sich die Funktionen des Messgeräts 112 und die des Spielzeugs 128 zum Messsystem 110 ergänzen. So kann, wie oben dargestellt, der Gesichtsbereich 204 beispielsweise ganz oder teilweise transparent ausgestaltet sein, so dass, beispielsweise durch eine transparente Abdeckfolie im Bereich der Öffnung 134, das Anzeigenelement 116 sichtbar sein kann.

Die Applikationsöffnung 124 kann ebenfalls in den Gesichtsbereich 204 gestalterisch integriert sein, so dass diese beispielsweise als "Mund" 208 wahrgenommen wird. Auch weitere Bestandteile des Messgeräts 112 können für einen Benutzer von außen sichtbar sein. So können beispielsweise Bereiche der Griffelemente 138, ebenfalls durch eine transparente Abdeckung, als "Backen" wahrnehmbar sein. Die beschriebenen Ausführungsformen stellen lediglich Beispiele dafür dar, wie ein handelsübliches Messgerät 112 gestalterisch in das Spielzeug 128 integriert werden kann. Zahlreiche weitere Ausführungsformen sind denkbar.

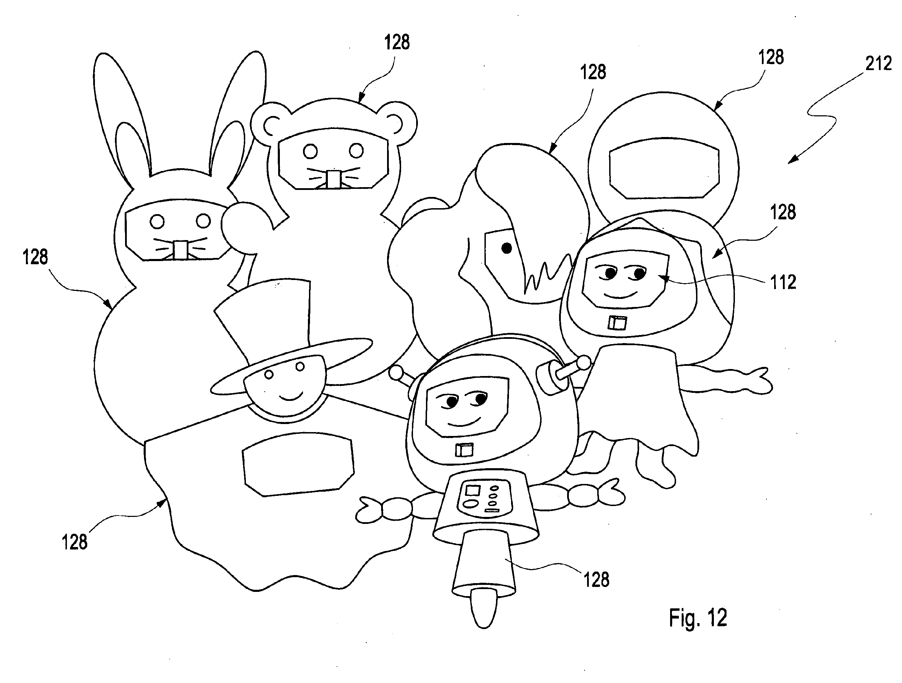

In Figur 12 ist schließlich symbolisch ein Ausführungsbeispiel eines erfindungsgemäßen Kits 212 dargestellt. Dieses Kit 212 kann einerseits zur Herstellung eines erfindungsgemäßen Messsystems 110 eingesetzt werden, kann jedoch andererseits auch von einem Benutzer als Kit zum Nachweis eines oder mehrerer Analyten in einer Körperflüssigkeit eingesetzt werden.

Das Kit umfasst neben einem oder mehreren Messgeräten 112, wobei vorzugsweise lediglich ein Messgerät 112 eingesetzt wird, eine Mehrzahl von Spielzeugen 128 in verschiedenen Ausführungsformen. Das Messgerät 112 ist in Figur 12 lediglich angedeutet. Jedes der Spielzeuge 128 ist eingerichtet, um ein Messgerät 112 aufzunehmen, beispielsweise gemäß der oben anhand der Figuren 5 und 6A und 6B ausgestalteten Ausführungsformen.

Die Spielzeuge 128 unterscheiden sich jedoch in ihrem äußeren Erscheinungsbild. So kann das Kit 212 beispielsweise eine Mehrzahl von Puppen, Fantasiefiguren, Spieltieren, insbesondere Kuscheltieren oder ähnlichen Spielzeugen 128 umfassen. Auch eine Integration in Spielzeuge 128, welchen die kindliche Fantasie keinen eigenen Charakter zuschreibt, ist grundsätzlich möglich.

Das gezeigte Kit 212 kann zu verschiedenen Zwecken eingesetzt werden. Zum einen erleichtern derartige Kits 212 die Produktion eines erfindungsgemäßen Messsystems 110. Die Mehrzahl von Spielzeugen 128 kann beispielsweise auf Lager gehalten werden, mit einem oder mehreren bestimmten Typen von Messgeräten 112. So kann, ohne größeren logistischen Aufwand, ein zielgruppenspezifisches Messsystem 110 auf einfache und kostengünstige Weise bereitgestellt werden. Alternativ oder zusätzlich kann ein Kit 212 jedoch auch von einem Benutzer oder einer Gruppe von Benutzern 162 eingesetzt werden, beispielsweise einer Familie. Auf diese Weise lassen sich die äußeren Erscheinungsbilder der Messsysteme 110 schnell auf den jeweiligen einzelnen Benutzer 162 anpassen, welcher eine Messung durchführen soll. Auch hierdurch lassen sich Kosten sparen, und die Benutzerfreundlichkeit lässt sich erheblich erhöhen.

### Bezueszeichenliste

- 110: Messsystem
- 112: Messgerät
- 114: Gehäuse
- 116: Anzeigenelement
- 118: Messwerte
- 120: Benutzungsanweisungen
- 122: Bedienelemente
- 124: Applikationsöffnung
- 126: Frontseite
- 128: Spielzeug
- 130: Spielzeughülle
- 132: Aufnahme
- 134: Öffnung
- 136: Öffnung
- 138: Griffelemente
- 140: Kopf
- 142: Aufnahmeraum
- 144: Aufnahmeöffnung
- 146: Verschlusselement
- 148: Reißverschluss
- 150: Öffnen des Verschlusselements
- 152: Einfügen des Messgeräts
- 154: Arme
- 156: Spielzeugsteuerung
- 158: Steuerung
- 160: Identifikator
- 162: Benutzer
- 164: Eingabesystem für akustische Signale
- 166: Ausgabesystem
- 168: Suchruf
- 170: Antwort
- 172: Testelement
- 174: akustische Signale
- 176: optische Signale
- 178: erste Kombination
- 180: Zeitanzeige
- 182: Erinnerungssignal
- 184: zweite Kombination
- 186: Warteanzeige
- 188: Warteaufforderung
- 190: dritte Kombination
- 192: vierte Kombination
- 194: positive Anzeige
- 196: positive akustische Bewertung
- 198: negative Anzeige
- 200: negative akustische Bewertung
- 202: Applikationsstelle
- 204: Gesichtsbereich
- 206: Ohren
- 208: Mund
- 210: Backen
- 212: Kit

## Patentansprüche

1. Messsystem (110) zum Nachweis mindestens eines Analyten in einer Körperflüssigkeit, insbesondere zum Nachweis von Blutglucose,
- umfassend mindestens ein Messgerät (112) zum Nachweis des Analyten in der Körperflüssigkeit, insbesondere ein handelsübliches Messgerät (112), wobei das Messgerät (112) als tragbares Handgerät ausgestaltet ist und ein Gehäuse (114) mit mindestens einem Anzeigenelement (116) aufweist, wobei das Messgerät (112) weiterhin mindestens ein Bedienelement (122) aufweist, wobei das Messgerät (112) eingerichtet ist, um den Analyten mittels mindestens eines Testelements (172) nachzuweisen, wobei das Messgerät (112) mindestens eine Applikationsöffnung (124) aufweist, wobei die Applikationsöffnung (124) eingerichtet ist, um eine Aufgabe einer Probe der Körperflüssigkeit zu ermöglichen,
- weiterhin umfassend ein Spielzeug (128), insbesondere eine Spielfigur oder ein Spieltier, wobei das Spielzeug (128) mindestens eine Aufnahme (132) zum reversiblen Aufnehmen des Messgeräts (112) aufweist, wobei die Aufnahme (132) derart eingerichtet ist, dass bei in der Aufnahme (132) aufgenommenem Messgerät (112) das Anzeigenelement (116) zumindest teilweise zugänglich ist, wobei die Aufnahme (132) derart eingerichtet ist, dass bei in der Aufnahme (132) aufgenommenem Messgerät (112) das Bedienelement (122) zumindest teilweise zugänglich ist, wobei die Aufnahme (132) derart eingerichtet ist, dass die Aufgabe der Probe bei in der Aufnahme (132) aufgenommenem Messgerät (112) durchführbar ist, wobei die Aufnahme (132) mindestens eine Fixiervorrichtung aufweist, wobei die Fixiervorrichtung eingerichtet ist, um das Messgerät (112) relativ zu dem Spielzeug (128) zu fixieren,
wobei das Messgerät (112) derart eingerichtet ist, dass dieses sowohl in dem Messsystem (110) als auch wahlweise unabhängig von dem Spielzeug (128) einsetzbar ist.

2. Messsystem (110) nach dem vorhergehenden Anspruch, wobei die Aufnahme (132) mindestens einen Aufnahmeraum (142) aufweist, wobei das Messgerät (112) in dem Aufnahmeraum (142) zumindest teilweise aufnehmbar ist, wobei die Aufnahme (132) weiterhin mindestens ein Verschlusselement (146) aufweist, wobei das Verschlusselement (146) eingerichtet ist, um das Messgerät (112) in einem geschlossenem Zustand in dem Aufnahmeraum (142) zu haltern und in einem geöffneten Zustand ein Herausnehmen des Messgeräts (112) aus der Aufnahme (132) zu ermöglichen.

3. Messsystem (110) nach dem vorhergehenden Anspruch, wobei das Verschlusselement (146) mindestens eines der folgenden Elemente umfasst: einen Reißverschluss (148); einen Druckknopf; einen Textilknopf; eine durch einen Benutzer (162) lösbare Sperre.

4. Messsystem (110) nach einem der vorhergehenden Ansprüche, wobei das Spielzeug (128) zumindest teilweise als menschliche Figur, als tierische Figur oder als Fantasiefigur ausgestaltet ist, wobei die Aufnahme (132) eine Öffnung (134, 136) aufweist, wobei die Öffnung (134, 136) zumindest teilweise in einem Gesichtsbereich der Figur angeordnet ist, wobei die Öffnung (134, 136) derart ausgestaltet ist, dass das Anzeigenelement (116) bei in der Aufnahme (132) aufgenommenem Messgerät (112) zumindest teilweise in der Öffnung (134, 136) erkennbar ist.

5. Messsystem (110) nach dem vorhergehenden Anspruch, wobei das Messgerät (112) eine Steuerung (158) aufweist, wobei die Steuerung (158) eingerichtet ist, um in zumindest einem Betriebsmodus zumindest Teile eines Gesichts der Figur auf dem Anzeigenelement (116) darzustellen.

6. Messsystem (110) nach dem vorhergehenden Anspruch, wobei die Steuerung (158) eingerichtet ist, um einen Gesichtsausdruck des Gesichts entsprechend eines oder mehrerer der folgenden Bedingungen zu verändern: eines Messergebnisses des Nachweises des Analyten in der Körperflüssigkeit; eines Zeitpunktes, insbesondere eines Zeitpunktes für eine durchzuführende Messung.

7. Messsystem (110) nach einem der vorhergehenden Ansprüche, wobei das Messgerät (112) weiterhin mindestens ein akustisches Element (166) aufweist, wobei das akustische Element (166) eingerichtet ist, um eine Ausgabe akustischer Signale, insbesondere eine Sprachausgabe, vorzunehmen.

8. Messsystem (110) nach dem vorhergehenden Anspruch, wobei die Ausgabe akustischer Signale, insbesondere die Sprachausgabe, eine oder mehrere der folgenden Funktionen umfasst: eine Ausgabe mindestens eines Messergebnisses des Nachweises des Analyten in der Körperflüssigkeit; eine Stellungnahme zu mindestens einem Messergebnis des Nachweises des Analyten in der Körperflüssigkeit; eine Aufforderung zur Vornahme einer Messung; eine Aufforderung zur Vornahme mindestens einer Handlung, insbesondere zum Austausch eines Energiespeichers; eine Antwort auf einen Suchruf eines Benutzers (162).

9. Messsystem (110) nach einem der vorhergehenden Ansprüche, wobei das Messgerät (112) mindestens ein Eingabeelement (164) zur Eingabe akustischer Signale umfasst.

10. Messsystem (110) nach einem der vorhergehenden Ansprüche, wobei das Messgerät (112) mindestens ein Speicherelement aufweist, wobei in dem Speicherelement eine Mehrzahl von Sätzen von Charakterparametern unterschiedlicher Arten von Spielzeugen (128) hinterlegt ist, wobei entsprechend des speziellen Spielzeuges (128) ein bestimmter Satz auswählbar ist.

11. Messsystem (110) nach einem der vorhergehenden Ansprüche, wobei das Spielzeug (128) mindestens eine Spielzeugsteuerung (156) aufweist, wobei die Spielzeugsteuerung (156) eingerichtet ist, um mindestens eine spielzeugspezifische Funktion durchzuführen, wobei das Spielzeug (128) weiterhin mindestens eine Schnittstelle zum Austausch von Daten und/oder Steuerbefehlen mit dem Messgerät (112) aufweist, wobei die Spielzeugsteuerung (156) eingerichtet ist, um mit einer Steuerung (158) des Messgeräts (112) zu kommunizieren.

12. Messsystem (110) nach dem vorhergehenden Anspruch, wobei das Messgerät (112) eingerichtet ist, um mindestens eine Funktion an das Spielzeug (128) anzupassen.

13. Messsystem (110) nach einem der vorhergehenden Ansprüche, wobei das Spielzeug (128) mindestens einen Identifikator (160) aufweist, wobei der Identifikator (160) mindestens eine Information über das Spielzeug (128) aufweist, wobei das Messgerät (112) eingerichtet ist, um den Identifikator (160) auszulesen, vorzugsweise automatisch.

14. Kit (212) zum Nachweis mindestens eines Analyten in einer Körperflüssigkeit, insbesondere zum Nachweis von Blutglucose, umfassend mindestens ein Messgerät (112) zum Nachweis des Analyten in der Körperflüssigkeit, insbesondere ein handelsübliches Messgerät (112), wobei das Messgerät (112) als tragbares Handgerät ausgestaltet ist und ein Gehäuse (114) mit mindestens einem Anzeigenelement (116) aufweist, weiterhin umfassend eine Mehrzahl von Spielzeugen (128), insbesondere Spielfiguren und/oder Spieltiere, wobei die Spielzeuge (128) jeweils mindestens eine Aufnahme (132) zum Aufnehmen des Messgeräts (112) aufweisen, wobei die Aufnahmen (132) derart eingerichtet sind, dass bei in der Aufnahme (132) aufgenommenem Messgerät (112) das Anzeigenelement (116) zumindest teilweise zugänglich ist, wobei das Messgerät (112) derart eingerichtet ist, dass dieses sowohl austauschbar in einem der Spielzeuge (128) als auch wahlweise unabhängig von den Spielzeugen (128) einsetzbar ist und wobei das Messgerät (112) und jeweils eines der Spielzeuge (128) zusammen ein Messsystem (110) nach einem der vorhergehenden Ansprüche bilden.

15. Verfahren zur Herstellung eines Messsystems (110) zum Nachweis mindestens eines Analyten in einer Körperflüssigkeit, insbesondere eines Messsystems (110) nach einem der vorhergehenden, ein Messsystem (110) betreffenden Ansprüche, wobei ein Messgerät (112) zum Nachweis des Analyten in der Körperflüssigkeit verwendet wird, insbesondere ein handelsübliches Messgerät (112), wobei das Messgerät (112) als tragbares Handgerät ausgestaltet ist und ein Gehäuse (114) mit mindestens einem Anzeigenelement (116) aufweist, wobei weiterhin ein Spielzeug (128) verwendet wird, insbesondere eine Spielfigur oder ein Spieltier, wobei das Messgerät (112) in mindestens einer Aufnahme (132) des Spielzeugs reversibel aufgenommen wird, wobei die Aufnahme (132) derart eingerichtet ist, dass bei in der Aufnahme (132) aufgenommenem Messgerät (112) das Anzeigenelement (116) zumindest teilweise zugänglich ist, wobei das Messgerät (112) weiterhin mindestens ein Bedienelement (122) aufweist, wobei die Aufnahme (132) derart eingerichtet ist, dass bei in der Aufnahme (132) aufgenommenem Messgerät (112) das Bedienelement (122) zumindest teilweise zugänglich ist, wobei das Messgerät (112) eingerichtet ist, um den Analyten mittels mindestens eines Testelements (172) nachzuweisen, wobei das Messgerät (112) mindestens eine Applikationsöffnung (124) aufweist, wobei die Applikationsöffnung (124) eingerichtet ist, um eine Aufgabe einer Probe der Körperflüssigkeit zu ermöglichen, wobei die Aufnahme (132) derart eingerichtet ist, dass die Aufgabe der Probe bei in der Aufnahme (132) aufgenommenem Messgerät (112) durchführbar ist, wobei die Aufnahme (132) mindestens eine Fixiervorrichtung aufweist, wobei die Fixiervorrichtung eingerichtet ist, um das Messgerät (112) relativ zu dem Spielzeug (128) zu fixieren, wobei das Messgerät (112) derart eingerichtet ist, dass dieses sowohl in dem Messsystem (110) als auch wahlweise unabhängig von dem Spielzeug (128) einsetzbar ist.

16. Verfahren nach dem vorhergehenden Anspruch, wobei in einer Lagerhaltung eine Mehrzahl von unterschiedlichen Arten von Spielzeugen (128) vorgehalten wird, wobei je nach Zielgruppe der Benutzer (162) eine bestimmte Art von Spielzeug (128) für die Herstellung des Messsystems (110) ausgewählt wird.

## Claims

1. Measurement system (110) for detecting at least one analyte in a bodily fluid, in particular for detecting blood glucose,
- comprising at least one measurement instrument (112) for detecting the analyte in the bodily fluid, in particular a commercially available measurement instrument (112), the measurement instrument (112) being designed as a portable hand-held instrument and having a housing (114) with at least one display element (116), in which the measurement instrument (112) furthermore has at least one operating element (122), in which the measurement instrument (112) is designed to detect the analyte using at least one test element (172), the measurement instrument (112) having at least one application opening (124), the application opening (124) being designed to enable a placement of a sample of the bodily fluid,
- furthermore comprising a toy (128), in particular a figurine or a toy animal, the toy (128) having at least one receptacle (132) for reversibly holding the measurement instrument (112), the receptacle (132) being designed such that if the measurement instrument (112) is held by the receptacle (132), the display element (116) is accessible, at least in part, the receptacle (132) being designed such that if the measurement instrument (112) is held by the receptacle (132), the operating element (122) is accessible, at least in part, with the receptacle (132) being designed such that the sample can be placed when the measurement instrument (112) is held in the receptacle (132), in which the receptacle (132) has at least one fixing apparatus, the fixing apparatus being designed to fix the measurement instrument (112) relative to the toy (128),
in which the measurement instrument (112) is designed such that it can be used both in the measurement system (110) and also, optionally, independently of the toy (128).

2. Measurement system (110) according to the preceding claim, in which the receptacle (132) has at least one receptacle space (142), it being possible for the measurement instrument (112) to be held at least in part in the receptacle space (142), with the receptacle (132) furthermore having at least one closing element (146), the closing element (146) being designed to secure the measurement instrument (112) in the receptacle space (142) when said closing element is in a closed state and to permit a removal of the measurement instrument (112) from the receptacle (132) when said closing element is in an opened state.

3. Measurement system (110) according to the preceding claim, in which the closing element (146) comprises at least one of the following elements: a zipper (148); a pushbutton; a textile button; a lock which can be released by a user (162).

4. Measurement system (110) according to one of the preceding claims, in which the toy (128) is at least in part designed as a human figure, an animal figure or a fantasy figure, with the receptacle (132) having an opening (134, 136), the opening (134, 136) being arranged at least in part in a facial region of the figure, the opening (134, 136) being designed such that the display element (116) can at least in part be recognized in the opening (134, 136) when the measurement instrument (112) is held in the receptacle (132).

5. Measurement system (110) according to the preceding claim, in which the measurement instrument (112) has a control (158), the control (158) being designed to display at least parts of a face of the figure on the display element (116) in at least one mode of operation.

6. Measurement system (110) according to the preceding claim, in which the control (158) is designed to change a facial expression of the face according to one or more of the following conditions: a measurement result of the detection of the analyte in the bodily fluid; a time, in particular a time for a measurement to be carried out.

7. Measurement system (110) according to one of the preceding claims, in which the measurement instrument (112) furthermore has at least one acoustic element (166), the acoustic element (166) being designed to output acoustic signals, in particular to output speech.

8. Measurement system (110) according to the preceding claim, in which the output of acoustic signals, in particular the speech output, comprises one or more of the following functions: an output of at least one measurement result of the detection of the analyte in the bodily fluid; a statement regarding at least one measurement result of the detection of the analyte in the bodily fluid; a request to undertake a measurement; a request to undertake at least one action, in particular exchanging an energy store; a reply to a search call of a user (162).

9. Measurement system (110) according to one of the preceding claims, in which the measurement instrument (112) comprises at least one input element (164) for inputting acoustic signals.

10. Measurement system (110) according to one of the preceding claims, in which the measurement instrument (112) has at least one storage element, with a multiplicity of sets of character parameters from different types of toys (128) being stored in the storage element, with it being possible for a certain record to be selected in accordance with the particular toy (128).

11. Measurement system (110) according to one of the preceding claims, in which the toy (128) has at least one toy control (156), the toy control (156) being designed to carry out at least one toy-specific function, the toy (128) furthermore having at least one interface for interchanging data and/or control commands with the measurement instrument (112), the toy control (156) being designed to communicate with a control (158) of the measurement instrument (112).

12. Measurement system (110) according to the preceding claim, in which the measurement instrument (112) is designed to adapt at least one function to the toy (128).

13. Measurement system (110) according to one of the preceding claims, in which the toy (128) has at least one identifier (160), the identifier (160) having at least one item of information about the toy (128), the measurement instrument (112) being designed to read out the identifier (160), preferably automatically.

14. Kit (212) for detecting at least one analyte in a bodily fluid, in particular for detecting blood glucose, comprising at least one measurement instrument (112) for detecting the analyte in the bodily fluid, in a particular a commercially available measurement instrument (112), the measurement instrument (112) being designed as a portable hand-held instrument and having a housing (114) with at least one display element (116), furthermore comprising a multiplicity of toys (128), in particular figurines and/or toy animals, the toys (128) each having at least one receptacle (132) for holding the measurement instrument (112), the receptacles (132) being designed such that if the measurement instrument (112) is held by the receptacle (132), the display element (116) is accessible, at least in part, in which the measurement instrument (112) is designed such that it can be used both interchangeably in one of the toys (128) and, optionally, independently of the toys (128) and in which the measurement instrument (112) and respectively one of the toys (128) together form a measurement system (110) according to one of the preceding claims.

15. Method for producing a measurement system (110) for detecting at least one analyte in a bodily fluid, in particular a measurement system (110) according to one of the preceding claims relating to a measurement system (110), in which a measurement instrument (112) is used to detect the analyte in the bodily fluid, in particular a commercially available measurement instrument (112), the measurement instrument (112) being designed as a portable hand-held instrument and having a housing (114) with at least one display element (116), in which furthermore a toy (128) is used, in particular a figurine or a toy animal, the measurement instrument (112) being held reversibly in at least one receptacle (132) of the toy, the receptacle (132) being designed such that if the measurement instrument (112) is held by the receptacle (132), the display element (116) is accessible, at least in part, in which the measurement instrument (112) furthermore has at least one operating element (122), the receptacle (132) being designed such that if the measurement instrument (112) is held by the receptacle (132), the operating element (122) is accessible, at least in part, in which the measurement instrument (112) is designed to detect the analyte using at least one test element (172), the measurement instrument (112) having at least one application opening (124), the application opening (124) being designed to enable the placement of a sample of the bodily fluid, with the receptacle (132) being designed such that the sample can be placed when the measurement instrument (112) is held in the receptacle (132), in which the receptacle (132) has at least one fixing apparatus, the fixing apparatus being designed to fix the measurement instrument (112) relative to the toy (128), in which the measurement instrument (112) is designed such that it can be used both in the measurement system (110) and, optionally, independently of the toy (128).

16. Method according to the preceding claim, in which a multiplicity of different types of toys (128) are stored in storage, with a certain type of toy (128) being selected to produce the measurement system (110) depending on the target group of the users (162).

## Revendications

1. Système de mesure (110) destiné à détecter au moins un analyte présent dans un liquide corporel et en particulier à détecter le glucose dans le sang,
- comprenant au moins un appareil de mesure (112) qui détecte l'analyte présent dans le liquide corporel, en particulier un appareil de mesure (112) présent sur le marché, l'appareil de mesure (112) étant configuré comme appareil manuel portable et présentant un boîtier (114) doté d'au moins un élément d'affichage (116), l'appareil de mesure (112) présentant en outre au moins un élément de commande (122), l'appareil de mesure (112) étant conçu pour détecter l'analyte au moyen d'au moins un élément de test (172), l'appareil de mesure (112) présentant au moins une ouverture d'application (124), l'ouverture d'application (124) étant conçue pour permettre de placer un échantillon du liquide corporel,
- comprenant en outre au moins un jouet (128), en particulier une figure de jeu ou un animal de jeu, le jouet (128) présentant au moins un logement (132) qui permet de loger l'appareil de mesure (112) de manière réversible, le logement (132) étant conçu de manière à donner un accès au moins partiel à l'élément d'affichage (116) lorsque l'appareil de mesure (112) est logé dans le logement (132), le logement (132) étant conçu de telle sorte que lorsque l'appareil de mesure (112) est logé dans le logement (132), l'élément de commande (122) soit au moins partiellement accessible, le logement (132) étant conçu de telle sorte que l'échantillon puisse être placé lorsque l'appareil de mesure (112) est logé dans le logement (132), le logement (132) présentant au moins un dispositif de fixation, le dispositif de fixation étant conçu pour immobiliser l'appareil de mesure (112) par rapport au jouet (128),
l'appareil de mesure (112) étant conçu de manière à pouvoir être utilisé sélectivement aussi bien dans le système de mesure (110) que de manière indépendante du jouet (128).

2. Système de mesure (110) selon la revendication précédente, dans lequel le logement (132) présente au moins un espace de réception (142), l'appareil de mesure (112) pouvant être reçu au moins en partie dans l'espace de réception (142), le logement (132) présentant en outre au moins un élément de fermeture (146), l'élément de fermeture (146) étant conçu pour maintenir l'appareil de mesure (112) lorsqu'il est en position fermée dans l'espace de réception (142) et pour permettre d'enlever l'appareil de mesure (112) du logement (132) lorsqu'il est en position ouverte.

3. Système de mesure (110) selon la revendication précédente, dans lequel l'élément de fermeture (146) comprend au moins l'un des éléments suivants : une fermeture à frottement (148), un bouton poussoir, un bouton textile ou un verrou qui peut être libéré par l'utilisateur (162).

4. Système de mesure (110) selon l'une des revendications précédentes, dans lequel le jouet (128) est configuré au moins en partie comme figure humaine, figure d'animal ou figure de fantaisie, le logement (132) présentant une ouverture (134, 136), l'ouverture (134, 136) étant disposée au moins en partie dans une zone de visage de la figure, l'ouverture (134, 136) étant configurée de telle sorte que l'élément d'affichage (116) puisse être vu au moins en partie dans l'ouverture (134, 136) lorsque l"appareil de mesure (112) est logé dans le logement (132).

5. Système de mesure (110) selon la revendication précédente, dans lequel l'appareil de mesure (112) présente une commande (158), la commande (158) étant conçue pour représenter sur l'élément d'affichage (116) au moins des parties du visage de la figure dans au moins un mode de fonctionnement.

6. Système de mesure (110) selon la revendication précédente, dans lequel la commande (158) est conçue pour modifier l'expression du visage en fonction d'une ou plusieurs des conditions suivantes : le résultat d'une mesure de détection de l'analyte dans le liquide corporel, un instant et en particulier l'instant auquel une mesure doit être exécutée.

7. Système de mesure (110) selon l'une des revendications précédentes, dans lequel l'appareil de mesure (112) présente en outre au moins un élément acoustique (166), l'élément acoustique (166) étant conçu pour délivrer des signaux acoustiques et en particulier une émission vocale.

8. Système de mesure (110) selon la revendication précédente, dans lequel l'émission de signaux acoustiques et en particulier l'émission vocale comprend une ou plusieurs des fonctions suivantes : la fourniture d'au moins un résultat de mesure de détection de l'analyte dans le liquide corporel, un avis sur au moins un résultat de mesure de détection de l'analyte dans le liquide corporel, une invitation à entreprendre une mesure, une invitation à entreprendre au moins une manipulation, en particulier à remplacer une réserve d'énergie et une réponse à un appel de l'utilisateur (162).

9. Système de mesure (110) selon l'une des revendications précédentes, dans lequel l'appareil de mesure (112) comporte au moins un élément d'introduction (164) qui permet d'introduire des signaux acoustiques.

10. Système de mesure (110) selon l'une des revendications précédentes, dans lequel l'appareil de mesure (112) présente au moins un élément de mémoire, plusieurs jeux de paramètres caractéristiques de différents types de jouets (128) étant conservés dans l'élément de mémoire, un jeu défini pouvant être sélectionné en fonction du jouet (128) particulier.

11. Système de mesure (110) selon l'une des revendications précédentes, dans lequel le jouet (128) présente au moins une commande de jouet (156), la commande de jouet (156) étant conçue pour exécuter au moins une fonction spécifique au jouet, le jouet (128) présentant en outre au moins une interface qui permet d'échanger des données et/ou des ordres de commande avec l'appareil de mesure (112), la commande (156) du jouet étant conçue pour communiquer avec une commande (158) de l'appareil de mesure (112).

12. Système de mesure (110) selon la revendication précédente, dans lequel l'appareil de mesure (112) est conçu pour adapter au moins une fonction au jouet (128).

13. Système de mesure (110) selon l'une des revendications précédentes, dans lequel le jouet (128) présente au moins un identificateur (160), l'identificateur (160) présentant au moins une information concernant le jouet (128), l'appareil de mesure (112) étant conçu pour lire l'identificateur (160), de préférence automatiquement.

14. Trousse (212) de détection d'au moins un analyte dans un liquide corporel, en particulier de détection du glucose présent dans le sang, et comprenant au moins un appareil de mesure (112) qui détecte l'analyte présent dans le liquide corporel, en particulier un appareil de mesure (112) habituel du marché, l'appareil de mesure (112) étant configuré comme appareil manuel portable et présentant un boîtier (114) doté d'au moins un élément d'affichage (116), et comprenant en outre plusieurs jouets (128), en particulier des figures de jeu et/ou des animaux de jeu, les jouets (128) présentant tous au moins un logement (132) qui loge l'appareil de mesure (112), les logements (132) étant conçus de manière à donner accès au moins en partie à l'élément d'affichage (116) lorsque l'appareil de mesure (112) est logé dans le logement (132), l'appareil de mesure (112) étant conçu de manière à pouvoir être utilisé sélectivement aussi bien de manière remplaçable dans l'un des jouets (128) qu'indépendamment des jouets (128), l'appareil de mesure (112) et chacun des jouets (128) formant ensemble un système de mesure (110) selon l'une des revendications précédentes.

15. Procédé de fabrication d'un système de mesure (110) destiné à détecter au moins un analyte présent dans un liquide corporel, en particulier un système de mesure (110) selon l'une des revendications précédentes qui concernent l'appareil de mesure (110), dans lequel on utilise un appareil (112) pour détecter l'analyte présent dans le liquide corporel, en particulier un appareil de mesure (112) habituel du marché, l'appareil de mesure (112) étant configuré comme appareil manuel portable et présentant un boîtier (114) doté d'au moins un élément d'affichage (116), un jouet (128), en particulier une figure de jeu ou un animal de jeu étant utilisé, l'appareil de mesure (112) étant reçu de manière réversible dans au moins un logement (132) du jouet, le logement (132) étant conçu de manière à donner au moins en partie accès à l'élément d'affichage (116) lorsque l'appareil de mesure (112) est logé dans le logement (132), l'appareil de mesure (112) présentant en outre au moins un élément de commande (122), l'appareil de mesure (132) étant conçu de manière à donner accès au moins en partie à l'élément de commande (122) lorsque l'appareil de mesure (112) est logé dans le logement (132), l'appareil de mesure (112) étant conçu pour détecter l'analyte au moyen d'au moins un élément de test (172), l'appareil de mesure (112) présentant au moins une ouverture d'application (124), l'ouverture d'application (124) étant conçue pour permettre de placer un échantillon du liquide corporel, le logement (132) étant conçu de manière à pouvoir réaliser le placement de l'échantillon lorsque l'appareil de mesure (112) est logé dans le logement (132), le logement (132) présentant au moins un dispositif de fixation, le dispositif de fixation étant conçu pour immobiliser l'appareil de mesure (112) par rapport au jouet (128), l'appareil de mesure (112) étant conçu pour pouvoir être utilisé aussi bien dans le système de mesure (110) que sélectivement de manière indépendante du jouet (128).

16. Procédé selon la revendication précédente, dans lequel plusieurs types différents de jouets (128) sont conservés dans une réserve, un type défini de jouet (128) étant sélectionné pour fabriquer le système de mesure (110) en fonction du groupe cible d'utilisateurs (162).
